# EUROPEAN PATENT APPLICATION

(11) **EP 4 635 565 A1**
(43) Date of publication of application: **22.10.2025**
(21) Application number: 24382406.7
(22) Date of filing: 17.04.2024
(51) Int. Cl.: A61P 9/00, A61K 31/47, A61P 19/02, A61P 25/00, A61P 35/00, C07D 215/20

(54) **PRENYLATED TETRAHYDROQUINOLINES AND QUINOLINES WITH PPAR AGONIST ACTIVITY**

(71) Applicant: Fundación para la Investigación del Hospital Clínico de la Comunidad Valenciana (INCLIVA), 46010 Valencia (ES); Universitat de València, 46010 Valencia (ES)
(72) Inventor: CABEDO ESCRIG, Nuria, Valencia (ES); VILLARROEL VICENTE, Carlos Luis, Valencia (ES); VILA DASÍ, Laura, Valencia (ES); MARTÍNEZ SOLSONA, María, Valencia (ES); CORTES MARTÍNEZ, Diego, Valencia (ES); GONZÁLEZ NAVARRO, Herminia, Valencia (ES); GARCÍA MARTÍN, Ainhoa Natividad, Valencia (ES); BERNABÉU SANCHÍS, Álvaro, Valencia (ES)
(74) Representative: Manuel Illescas y Asociados, S.L.U.

(57) **Abstract**

Prenylated tetrahydroquinolines and quinolines and pharmaceutical compositions comprising the same. Prenylated tetrahydroquinoline and quinolines and pharmaceutical compositions comprising the same, for use in the prevention and/or treatment of peroxisome proliferator-activated receptor (PPAR)-mediated diseases such as metabolic syndrome, type 2 diabetes mellitus, dyslipidemia, hyperlipidemia, hypertriglyceridemia, hypercholesterolemia, obesity, dyslipidemic atherosclerosis, metabolic dysfunction-associated fatty liver disease (MAFLD), cardiovascular disease, cardiometabolic disease, neurodegenerative disease, Friedreich's ataxia, Parkinson's disease, multiple sclerosis, Alzheimer's disease, autoimmune disease, rheumatoid arthritis, autoimmune thyroid disease, dermatological disease, and cancer.

## Description

### TECHNICAL FIELD

The present invention refers to prenylated tetrahydroquinoline compounds or prenylated quinoline compounds and to pharmaceutical compositions comprising the same and to their use in the prevention or treatment of peroxisome proliferator-activated receptor (PPAR)-mediated diseases.

### BACKGROUND ART

The peroxisome proliferator-activated receptors (PPARs) are nuclear receptors capable of regulating glucose homeostasis, lipid metabolism, inflammatory processes, cell differentiation processes and mitochondrial biogenesis, among others. These receptors are therapeutic targets for the treatment of cardiometabolic diseases, metabolic syndrome, type 2 diabetes mellitus, hyperlipidemias, dyslipidemic atherosclerosis, obesity, metabolic dysfunction-associated fatty liver disease (MAFLD), neurodegenerative diseases, Friedreich's ataxia, Parkinson's disease, Alzheimer's disease, autoimmune diseases, rheumatoid arthritis, autoimmune thyroid disease, dermatological diseases, and cancer. Three isoforms of PPARs have been identified: PPARα, PPARδ and PPARγ. PPARδ is also known as PPARβ.

Selective PPARα agonists of the fibrates group (fenofibrate, gemfibrozil, fenofibric acid, bezafibrate, etofibrate and ciprofibrate), are used for the treatment of dyslipidemias (hypertriglyceridemia, hypercholesterolemia).

Glitazone agonists (rosiglitazone, pioglitazone and troglitazone) are potent PPARγ-selective agonists for the treatment of type 2 diabetes, but with important clinical limitations. Potent activation of PPARγ has been associated with the development of serious adverse effects (cardiovascular risk, hepatotoxicity and bladder cancer) leading to the market removal of troglitazone in several countries, and to the market removal of rosiglitazone in the European Union. In several countries, clinical use of pioglitazone has been limited. In the United States, clinical use of rosiglitazone has been limited.

Glitazar agonists (aleglitazar, muraglitazar, tesaglitazar, and saroglitazar) are dual PPARa/y agonists for treatment of metabolic syndrome. Aleglitazar, muraglitazar, tesaglitazar have been removed from the market due to the adverse effects associated with their potent PPARγ agonism. Saroglitazar has been approved in India for the treatment of metabolic syndrome.

The pan-PPAR agonist lanifibranor is currently under a phase III clinical trial in China for the treatment of non-alcoholic fatty liver disease.

The prior art document EP3733658 from the inventors discloses the dual PPARα/γ agonist activity of polycerasidol and polycerasoidin, prenylated benzopyran natural compounds from plants, and discloses several synthetic prenylated benzopyran compounds with dual PPARa/y or pan-PPAR agonist activity.

There is a need to develop new PPAR agonists, to increase the treatment options for the patients, provide safer, more effective and/or better benefit/risk ratio alternatives, and make it possible to select therapies according to the patient's profile.

### SUMMARY OF INVENTION

As used herein, the term "alkyl" refers to a hydrocarbon chain, fully saturated, that can be linear or branched.

As used herein, the term "allyl" refers to a hydrocarbon chain, linear or branched, which comprises at least one -CH=CH-CH₂- group.

As used herein, the term "alkylamide" refers to a hydrocarbon chain, linear or branched, substituted with an amide group.

As used herein, the term "alkylamine" refers to a hydrocarbon chain, linear or branched, substituted with an amine group.

As used herein, the term "alkylaryl" refers to a hydrocarbon chain substituted with an aryl group, as defined below.

As used herein, the term "aryl" refers to a group derived from an aromatic hydrocarbon formed by removing one hydrogen atom from an aromatic ring in said aromatic hydrocarbon. As used herein, said aryl groups can be substituted.

As used herein, the term "alkyl-ether" refers to a saturated hydrocarbon chain substituted with an alkoxide group, as defined below.

As used herein, the term "alkoxide" refers to an oxygen atom linked to a hydrocarbon chain, where said hydrocarbon chain may comprise an alkyl and/or an aryl group, as defined above.

As used herein, the term "acyl" refers to a carbonyl group linked to a hydrocarbon chain, where said hydrocarbon chain may comprise an alkyl and/or an aryl group, as defined above.

As used herein, the expression "effective amount" refers to a quantity of a compound, salt, solvate or pharmaceutical composition of the invention sufficient to achieve a desired effect, for example, in a subject being administered with said compound, salt, solvate or pharmaceutical composition of the invention for prevention and/or treatment of a disease. An effective amount may be an amount sufficient to prevent or treat a disease in a subject. An effective amount may be an amount sufficient to reduce or ameliorate one or more symptoms of a disease in a subject. The effective amount (for example an amount treating, preventing, and/or ameliorating a disease in a subject) will be dependent on, for example, the particular disease being treated, the subject, and the route of administration.

The compounds of the present invention differ from those of EP3733658 in that they have a quinoline or tetrahidroquinoline core, which is a structurally distant core with respect to the benzopyran core of the compounds of EP3733658.

The problem to be solved consists in providing improved or alternative compounds for the treatment of PPAR-mediated diseases.

The present invention solves this problem by providing prenylated tetrahydroquinoline compounds or prenylated quinoline compounds, of Formula I or Formula II, respectively.

Compared with benzopyrans of EP3733658, the compounds of the present invention, advantageously, decrease levels of total cholesterol in plasma, decrease levels of non-HDL cholesterol ("bad cholesterol") in blood and decrease the HOMA-IR index (Homeostasis Model Assessment - Insulin Resistance), and increase the expression of genes involved in lipid metabolism and energy expenditure, such as the *Pdk4* gene in liver and fat, and *Acox1* and *Cpt1a* in mouse fat.

Furthermore, unlike benzopyrans, quinolines and tetrahydroquinolines are nitrogenous heterocycles with a weak basicity and can form salts in the presence of acids, improving bioavailability in the formulation of medications that contain them.

The present invention provides a prenylated tetrahydroquinoline compound or prenylated quinoline compound, of Formula I or Formula II, respectively, or a salt, or solvate thereof: 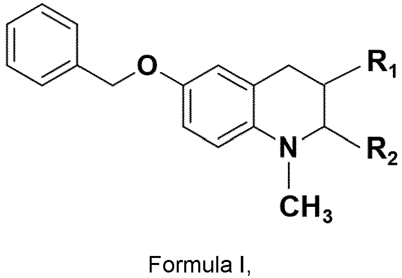 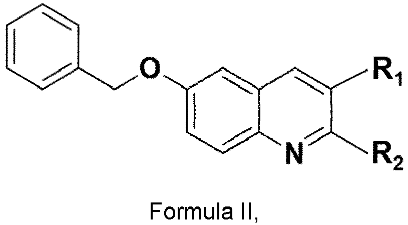 wherein:
- R₁ is independently selected from H or CH₂-CH=C(CH₃)-CH₂-CH₂-COOR₃;
- R₂ is independently selected from CH₃ or CH₂-CH₂-CH=C(CH₃)-CH₂-CH₂-COOR₃;
- R₃ is independently selected from the group consisting of H, alkyl, alkylamide, alkylamine, alkylaryl, alkyl-ether, and acyl; and
   wherein:
   - when R₁ is H, then R₂ is CH₂-CH₂-CH=C(CH₃)-CH₂-CH₂-COOR₃; and
   - when R₂ is CH₃, then R₁ is CH₂-CH=C(CH₃)-CH₂-CH₂-COOR₃.

In embodiments of the compound, salt, or solvate of the present invention, the alkyl is a C₁₋₆ alkyl.

In embodiments of the compound, salt, or solvate of the present invention, R₃ is independently selected from H or C₁₋₆ alkyl.

In embodiments of the compound, salt, or solvate of the present invention, R₃ is independently selected from H or CH₂-CH₃.

The compounds of the present invention have pan-PPAR agonist activity, dual PPARα/γ agonist activity, PPARα/δ agonist activity or selective PPARα agonist activity, regulate inflammation and have low or no cytotoxicity.

In embodiments of the compound, salt, or solvate of the present invention, said compound is selected from the group consisting of 5a, 5b, 6a, 6b, 8a, 8b, 9a and 9b: 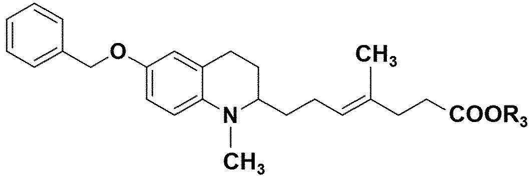
5a: R₃ = CH₂CH₃,
6a: R₃ = H,

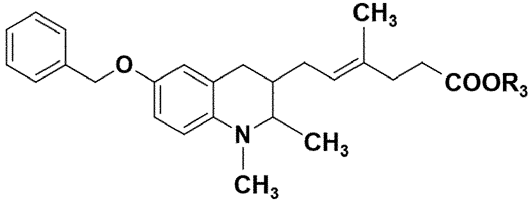

5b: R₃ = CH₂CH₃,
6b: R₃ = H,

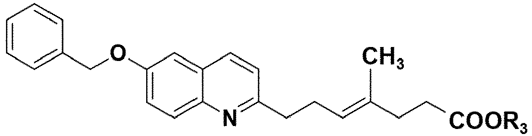

8a: R₃ = CH₂CH₃,
9a: R₃ = H,

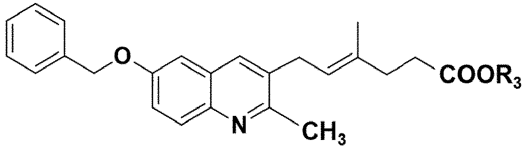

8b: R₃ = CH₂CH₃,
9b: R₃ = H.

The present invention also provides a pharmaceutical composition comprising at least a compound, salt, or solvate of the invention as active ingredient, and at least a pharmaceutically acceptable excipient or carrier.

In embodiments, the pharmaceutical composition of the invention further comprises at least one additional active ingredient.

The pharmaceutically acceptable excipient or carrier is selected, without limitation, from the group consisting of diluent, thickener, buffer, binder, disintegrant, coating agent, filler, lubricant, glidant, sweetener, flavour, preservative and surfactant.

The pharmaceutical composition of the invention may be administered to a subject by a route of administration selected, without limitation, from the group consisting of oral, ocular, topical, transdermal, intranasal, rectal, inhalation, parenteral, intradermal, subcutaneous, intramuscular, intraperitoneal, intra-arterial, intralymphatic, intravenous, intrathecal, intracranial and intratracheal.

The form of the pharmaceutical composition of the invention is selected, without limitation, from the group consisting of crystalline, powder, granular, compacted solid, liquid, solution, suspension, elixir, syrup, emulsion, cream, gel, droplet, mist, vapor and spray. The pharmaceutical composition of the invention may be formulated as, or be contained in, without limitation, from the group consisting of capsule, tablet, pill, caplet, ampoule, sachet, syringe, cartridge, and nebulizer.

The pharmaceutical composition of the invention may be administered in single or multiple doses.

The pharmaceutical composition of the invention may be formulated to provide controlled release of the active ingredient such as sustained or prolonged release.

In embodiments, the compound, salt, solvate or pharmaceutical composition of the present invention is for use as a medicament.

In embodiments, the present invention provides the use of the compound, salt, solvate, or pharmaceutical composition of the present invention for the preparation of a medicament.

In embodiments, the compound, salt, solvate, or pharmaceutical composition of the present invention is for use in the prevention and/or treatment of a peroxisome proliferator-activated receptor (PPAR)-mediated disease.

In embodiments, the present invention provides a method for preventing and/or treating a peroxisome proliferator-activated receptor (PPAR)-mediated disease, said method comprising administering an effective amount of the compound, salt, solvate, or pharmaceutical composition of the present invention, to a subject in need thereof.

In embodiments, the present invention provides the use of the compound, salt, solvate, or pharmaceutical composition of the present invention for the preparation of a medicament for the prevention and/or treatment of a peroxisome proliferator-activated receptor (PPAR)-mediated disease.

In embodiments of the compound, salt, solvate, or pharmaceutical composition for use of the present invention, said compound is an agonist of at least one PPAR protein, wherein said PPAR protein is selected from the group consisting of PPARα, PPARβ/δ and PPARγ.

In embodiments of the compound, salt, solvate, or pharmaceutical composition for use of the present invention, said compound is a PPARγ agonist with partial PPARγ agonist activity. The effects of PPARγ agonists with partial PPARγ agonist activity of the invention are safety improvement and avoidance of the adverse effects associated with this isoform (PPARγ).

As used herein, rosiglitazone is considered a PPARγ agonist with full PPARγ agonist activity. As used herein, the term "partial PPARγ agonist activity" refers to a PPARγ agonist activity from 15% to 85% of the activity of the rosiglitazone.

In embodiments of the compound, salt, solvate, or pharmaceutical composition for use of the present invention, said compound is a dual PPARα/γ agonist. The effect of dual PPARα/γ agonists of the invention is more effectivity in the treatment of metabolic diseases, reduction the risk of atherosclerosis associated with metabolic diseases, and lower toxicity.

In embodiments of the compound, salt, solvate, or pharmaceutical composition for use of the present invention, said compound is an agonist of PPARα, PPARβ/δ and PPARγ (a pan-PPAR agonist). The effect of pan-PPAR agonists of the invention is more effectivity in the treatment of metabolic diseases, reduction the risk of atherosclerosis associated with metabolic diseases, and lower toxicity.

In embodiments of the compound, salt, solvate, or pharmaceutical composition for use of the present invention, said disease is selected from the group consisting of PPARα-mediated disease, PPARβ/δ-mediated disease, and PPARγ-mediated disease.

In embodiments of the compound, salt, solvate, or pharmaceutical composition for use of the present invention, said disease is associated to the expression of a gene selected from the group consisting of *Pdk4*, *NF-κB*, *CcI2*, *CcI5*, *Il6*, *Tnf, Cpt1a,* and *Acox1.*

The compounds of the invention regulate neuroinflammation and autoimmune inflammatory processes and has therapeutic potential for neurodegenerative diseases such as Friedreich's ataxia, Parkinson's disease, Alzheimer's disease and multiple sclerosis.

Therefore, in embodiments of the compound, salt, solvate, or pharmaceutical composition for use of the present invention, said disease is associated with inflammation. Preferably, said inflammation is selected from autoimmune inflammation or neuroinflammation.

In embodiments of the compound, salt, solvate, or pharmaceutical composition for use of the present invention, said wherein said disease is selected from the group consisting of metabolic syndrome, type 2 diabetes mellitus, dyslipidemia, hyperlipidemia, hypertriglyceridemia, hypercholesterolemia, obesity, dyslipidemic atherosclerosis, metabolic dysfunction-associated fatty liver disease (MAFLD), cardiovascular disease, cardiometabolic disease, neurodegenerative disease, Friedreich's ataxia, Parkinson's disease, multiple sclerosis, Alzheimer's disease, autoimmune disease, rheumatoid arthritis, autoimmune thyroid disease, dermatological disease, and cancer.

The examples of the present invention show pan-PPAR agonist activity of the compounds of the invention **5a**, **6a**, and **8a** and dual PPARα/γ agonist activity of the compounds of the invention **9a**, **8b**, and **9b**, always with partial activity on the PPARγ isoform. The 3-substituted tetrahydroquinoline compounds of the invention **5b** and **6b** were selective PPARα, with efficiencies 3-4 times higher than the prior art compound WY-14,643. All compounds showed low or no toxicity at the dose of 100 µM and 30 µM, respectively, in human THP-1 cells differentiated into macrophages. *In vitro* studies revealed that tetrahydroquinoline compounds of the invention **5a** and **5b** and quinoline compound of the invention **8a** promote lipid metabolism and minimize inflammation, as they increased the transactivation of the PDK4-Luc+ indicator and the expression of the *Pdk4* gene, repressed the transactivation of NF-κB-TNFα-dependent Luc+ and downregulated the transcription of several TNFα-induced proinflammatory genes in L929sA WT cells at the dose of 10 µM. Regarding *in vivo* studies in preclinical models, tetrahydroquinoline compound of the invention **5a** administered (orally, 10 mg/kg/d for 15 days) to an obese and diabetic mouse model (*ob*/*ob*)*,* improved lipid and carbohydrate parameters. These changes consisted of a decrease in total cholesterol and non-HDL cholesterol levels, as well as insulin levels and the HOMA-IR index in the blood of *ob*/*ob* mice treated with tetrahydroquinoline compound of the invention **5a** compared to mice treated with vehicle. The tetrahydroquinoline compound of the invention **5a** also increased the expression of genes involved in lipid metabolism and energy expenditure, such as the *Pdk4* gene in liver and fat, and *Acox1* and *Cpt1a* in *oblob* mouse fat.

Unless defined otherwise, all the technical and scientific terms used herein have the same meaning as those commonly understood by a person skilled in the art in the field of the invention.

The term "comprise", "comprising" and their variants, throughout the description and the claims, includes, specifically, the term "consisting" or "consisting of".

As used in the description and the claims, the singular forms "a", "an", and "the" include plural referents unless the content clearly dictates otherwise.

### BRIEF DESCRIPTION OF DRAWINGS

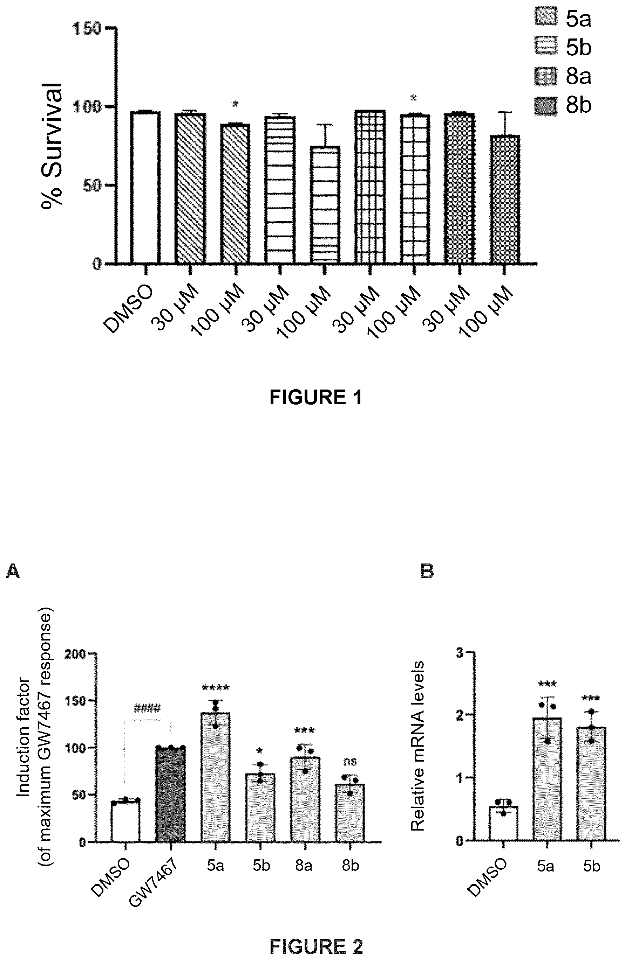

**Figure 1****.** Effects of tetrahydroquinoline compounds **5a** and **5b**, and quinoline compounds **8a** and **8b** on the viability of human macrophage cell line. THP-1 cells were treated with concentrations of 30 and 100 µM for 24 h and then cell lysates were processed with an Annexin V-assay kit according to the manufacturer's instructions, using flow cytometry. Data are presented as mean ± SEM of *n* = 3 independent experiments. *p < 0.05, relative to vehicle group.
**Figure 2****. Effects of tetrahydroquinolines and quinolines on PPAR target gene *Pdk4.*** (**A**) L929sA cells stably transfected with PDK4-(PPRE)₃-Luc+ and a constitutive β-gal expressing plasmid, were treated with solvent, compound GW7647 (1 µM), **5a**, **5b**, **8a**, or **8b** (10 µM) for 6 h. Cell lysates were assayed for luciferase activity and normalised to β-gal activity and expressed as the relative induction (% of maximal GW7647 response). (**B**) L929sA WT cells were incubated with solvent, **5a** or **5b** (10 µM) for 6 h. mRNA was isolated, reverse-transcribed, and subjected to RT-qPCR using primers to detect *Pdk4.* Results were normalised to household genes. RT-qPCR measurements were performed in triplicates. (ns, non-significant; **p* < 0.05, ****p* < 0.001, *****p* < 0.0001; *^{####}p* < 0.0001).

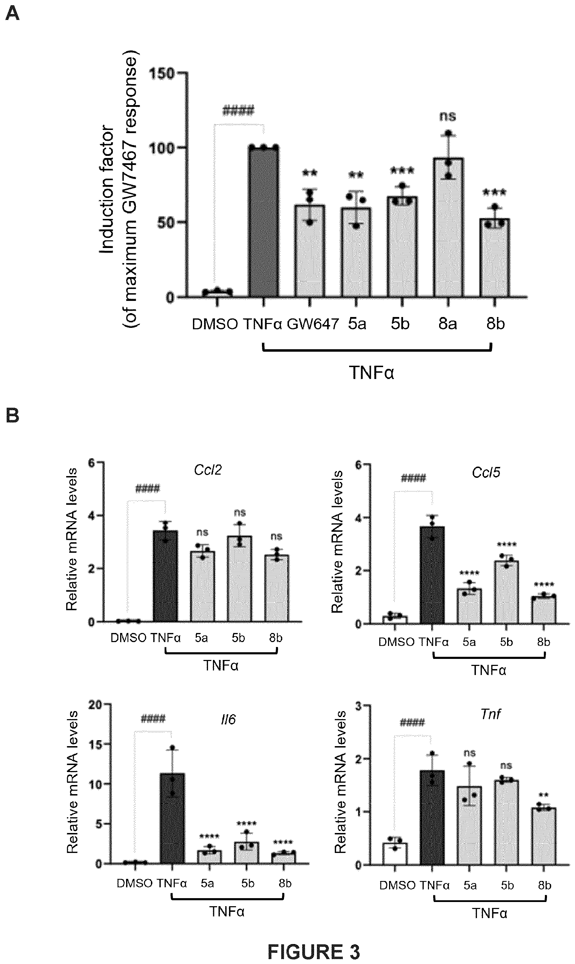

**Figure 3****.** Evaluation of anti-inflammatory effects of PPAR agonists. (**A**) L929 cells stably transfected with NF-κB-IL8P-Luc+ were incubated with solvent, compound GW7647 (1 µM), **5a**, **8a**, **5b** or **8b** (10 µM), for 1 h, before TNF (2000 IU/ml) was added for a total induction time of 6 h. Cell lysates were assayed for luciferase activity, normalised to β-gal activity and expressed as the relative induction (% of maximal TNFα response). Results were obtained from three independent biological replicates (*n*=3) with duplicate measurements. (**B**) L929sA WT cells were incubated with solvent, **5a**, **5b**, or **8b** (10 µM) for 1 h, before TNF (2000 IU/ml) was added, for a total induction time of 6 h. mRNA was isolated, reverse-transcribed, and subjected to RT-qPCR using primers to detect *CcI2, CcI5, Il6*, and *Tnf.* Results were normalised to household genes. RT-qPCR measurements were obtained from three independent biological replicated (*n*=3) and performed in triplicates.

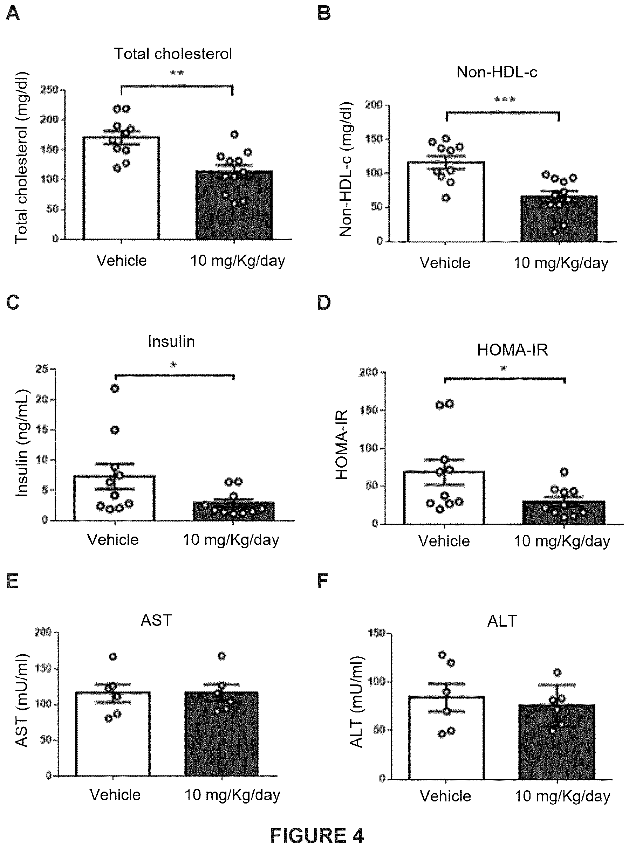

**Figure 4****.** Biochemical parameters in blood of *ob*/*ob* mice treated with tetrahydroquinoline **5a** (10 mg/kg/d, orally, 15 days) or vehicle. Tetrahydroquinoline **5a** reduces the plasma levels of total cholesterol (**A**), non-HDL-c (**B**), insulin (**C**), and HOMA-IR index (**D**) in *oblob* mice. Tetrahydroquinoline **5a** does not increase AST hepatic transaminase (**E**) and ALT hepatic transaminase (**F**) in *ob*/*ob* mice. Note: non-HDL-c (mg/L) = total cholesterol (mg/L) - HDL-c (mg/L); HOMA-IR index= fasting glucose (mmol/L) × fasting insulin (mU/mL)]/22.5. Values are presented as mean ± SEM of 5-12 independent experiments. *P < 0.05 relative to vehicle-treated mice.

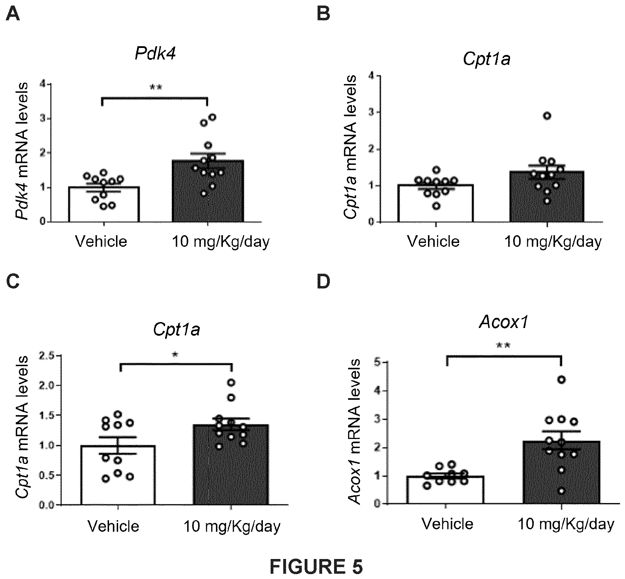

**Figure 5**. Treatment with tetrahydroquinoline **5a** (10 µM) modulates the expression of relevant PPARs target genes in the liver and epididymal fat of *ob*/*ob* mice. mRNA expression of *Pdk4* (**A**) and *Cpt1a* (**B**) in liver were analysed by RT-PCR. mRNA expression of *Cpt1a* (**C**) and *Acox1* (**D**) in epididymal white adipose tissue were analysed by RT-PCR. The mRNA levels were normalized to the expression of the constitutive cyclophilin gene. Values are presented as mean ± SEM of 5 independent experiments. *P < 0.05 relative to vehicle-treated mice.

### DESCRIPTION OF EMBODIMENTS

### Example 1. Materials and methods

### Analytical chemistry

High-resolution mass spectrometry electrospray ionization (HRMS(ESI)) data were collected on a TripleTOF^{™} 6600 (AB SCIEX). Liquid chromatography-mass spectrometry was performed using a liquid chromatography (UHPLC) platform (Shimadzu, LCMS-8040) coupled with a tandem mass spectrometry (MS/MS) triple quadrupole mass spectrometer equipped with an electrospray ionization (ESI) source (Shimadzu, Kyoto, Japan). ¹H and ¹³C NMR spectra, COSY 45 and HSQC were recorded on a Bruker Avance III 300 spectrometer, with CDCl₃ or CDCl₃ + 1 drop CD₃OD as solvent. Chemical shifts (δ) are reported in ppm relative to the internal reference deuterated solvent, with multiplicities indicated as s (singlet), d (doublet), t (triplet), q (quartet) and m (multiplet). The coupling constants (*J*) are expressed in hertz (Hz). All reactions were monitored by analytical thin-layer chromatography with silica gel 60 F₂₅₄ (Merck 5554; Merck Group, Darmstadt, Germany). The residues were purified by silica gel column chromatography (40-63 µm, Merck Group). The solvents and reagents were purchased from Scharlab S.L. (Barcelona, Spain) and Sigma-Aldrich (St. Louis, MO, USA) and used without further purification unless otherwise stated. Dry and freshly distilled solvents were used in the reactions carried out under N₂ atmosphere. Quoted yields were obtained from the purified materials. The final compounds were purified to ≥95% purity, as assessed by ¹H NMR and LC-MS/MS.

### Cell cultures

THP-1 cells were maintained under standard culture conditions (Dulbecco's modified Eagle's medium (DMEM) supplemented with 10% fetal calf serum (FCS) (both from Thermo Fisher Scientific, Waltham, MA, USA) at 37 °C in a 5% CO₂ humidified atmosphere. The medium was changed every two days. L929sA cells were grown in DMEM supplemented with 10% fetal calf serum, 100 U/mL penicillin and 0.1 mg/mL streptomycin. The cells were maintained in a humidified atmosphere containing 5% CO₂ at 37 °C.

### Plasmids and reagents

PDK4-(PPRE)₃-Luc+ reporter gene construct was generated by amplification of the PPAR response elements (PPRE) peak sequences in the *Pdk4* enhancer region from mouse liver genomic DNA and ligation in a pGL3-basic vector (Promega Biotec, Madison, Wisconsin). The p1481.IL8P-Luc+ reporter gene construct contains an IL-8 promoter fragment with one *NF*-*κB*-binding element. Compounds WY-14,643, GW7647, GW501516 and rosiglitazone were purchased from Sigma-Aldrich. **Table 1** discloses chemical data of said compounds. Recombinant murine TNFa was produced and purified. TNFa was used at a final concentration of 2000 IU/ml, where indicated.

**Table 1**

| **Compound** | |
|---|---|
| WY-14,643 | **Synonyms** |
| | pirinixic acid; 2-({4-chloro-6-[(2,3-dimethylphenyl)amino]pyrimidin-2-yl}sulfanyl)acetic acid |
| | **Chemical formula** |
| | 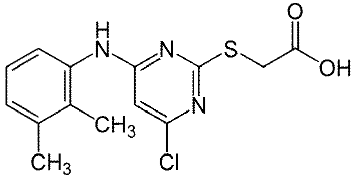 |
| GW7647 | **Synonym** |
| | 2-[(4-{2-[(4-Cyclohexylbutyl)(cyclohexylcarbamoyl)amino]ethyl}phenyl)sulfanyl]-2-methylpropanoic acid |
| | **Chemical formula** |
| | 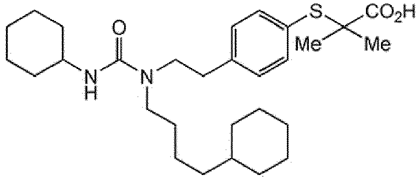 |
| GW501516 | **Synonyms** |
| | cardarine; 2-methyl-4-[({4-methyl-2-[4-(trifluoromethyl)phenyl]-1,3-thiazol-5-yl}methyl)sulfanyl]-2-methylphenoxy}acetic acid |
| | **Chemical formula** |
| | 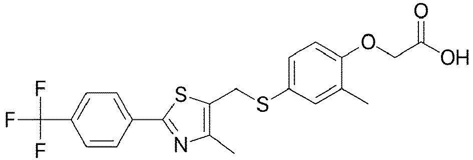 |
| Rosiglitazone | **Synonym** |
| | 5-[[4-[2-[methyl(pyridin-2-yl)amino]ethoxy]phenyl]methyl]-1,3-thiazol idine-2,4-dione |
| | **Chemical formula** |
| | 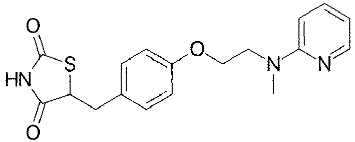 |

### PPAR transactivation assays

PPAR transcriptional activity was monitored using a human chimeric PPAR/Gal4 reporter luciferase system in Cos-7 cells (CRL-1651; ATCC, Manassas, VA, USA), transiently transfected with a luciferase reporter plasmid in the presence of expression vectors of pGAL4hPPARα, pGAL4hPPARβ/δ, and pGAL4hPPARγ. The responses of the compounds of the invention were compared with those of the following prior art compounds: WY-14,643 for PPARα, GW501516 for PPARβ/δ, and rosiglitazone for PPARγ. EC₅₀ was determined using a concentration range of 0.001-10 µmol/L. Luciferase activity was normalised to that of the internal control, β-galactosidase. Cells (5.5 × 10⁵ cells/mL) were seeded in 60-mm dishes in DMEM/10% FCS and incubated at 37 °C in a 5% solution for 16 h prior to transfection. Cells were transfected in DMEM with the jetPEI transfection reagent (Polyplus-Transfection S.A., Strasbourg, France) using the reporter plasmid pG5-TK-pGL3 in combination with one of the following expression plasmids: pGal4hPPARα, pGal4hPPARβ/δ, or pGal4hPPARy. The pCMV-β-galactosidase expression plasmid was used as a control for the transfection efficiency. Transfections were stopped after 16 h by adding DMEM/10% FCS. Cells were detached with trypsin, re-seeded in 96-well plates for 6 h in DMEM/10% FCS, and incubated for 24 h in DMEM/0.2% FCS with increasing concentrations of the test compounds or vehicle (DMSO, 0.1% final concentration). At the end of the experiment, cells were washed once with ice-cold PBS, lysed, and luciferase and β-galactosidase activities were measured. Luciferase activity was normalised to that of the internal control, β-galactosidase. All transfection experiments were performed at least five times.

### Cytotoxicity studies

The cytotoxicity of tetrahydroquinoline and quinoline esters **5a**, **5b**, **8a** and **8b** was evaluated at 30 and 100 µM by flow cytometry to assess cell survival using an Annexin V-FITC/PI dual staining assay. THP-1 cells (3 × 10⁴ cells per well) were seeded in 24-well plates and allowed to grow overnight. The medium was then replaced and incubations with the different compounds or DMSO were performed during 24 h. Then, all cells were processed with the Annexin V-assay kit (ANXCFK7, Immunostep, Salamanca, Spain) according to the manufacturer's instruction. A Becton Dickinson LSR Fortessa cytometer (BD Biosciences, Franklin Lakes, NJ, USA) was used for samples, and cell analysis were performed using FACSDiva X20 software.

### Pdk4 transactivation assays

Transfection of L929sA cells stably expressing reporter genes was performed using the calcium phosphate precipitation protocol according to standard procedures with a 10-fold excess of the plasmid of interest in a pGL3-basic vector containing Luc+, a modified firefly luciferase gene (Promega Biotec, Madison, Wis,) USA. USA). The *Pdk4* reporter was created using an enhancer site linked to the *Pdk4* promoter containing three PPRE extracted from mouse liver genomic DNA. The aforementioned plasmid construct was then introduced into a pMet7 backbone, which had previously been cloned with a luciferase gene site, using Xhol and Sacl cut sites. The resulting construct was subjected to geneticin (neomycin) antibiotic selection to isolate individual clones. A stable cell line, designated Pdk4-Luc+, was subsequently established in L929sA cells, which also contained a constitutively expressed galactosidase reporter and a neomycin selection cassette.

### NF-κB-IL8P transactivation assays

L929sA mouse fibroblasts were stably transfected using the calcium phosphate precipitation protocol, as described previously with p1481.IL8P-Luc+ plasmid construct. The indicated induction was performed at least in triplicates. All conditions were solvent-controlled. Subsequently, the cells were washed with PBS and lysed using TROPIX lysis buffer. Cellular luciferase levels were normalised to those of β-galactosidase (TROPIX, Bedford, MA, USA).

### Determination of mRNA expression by quantitative RT-qPCR

RNA was isolated using the RNeasy Purification Kit (Qiagen) according to the manufacturer's instructions. cDNA was synthesised using a PrimeScript kit (Takara). RT-qPCR was performed using Light Cycler 480 SYBR Green I Master Mix (Roche). RT-qPCR data were normalised and quantified relative to the two most stable reference genes.

### In vivo studies

Animal studies were carried out in accordance with Directive 2010/63/EU for experimentation on animals. The handling of the animals and the experimental protocols were approved by the Animal Experimentation Ethics Committee of the University of Valencia (Protocol No. A1469445706311). Six-week-old male *ob*/*ob* mice (C57BL/6.Cg-Lepob/J) were supplied by Charles River Laboratories International (Wilmington, MA). Blood samples were collected from mice fasted for 6 hours to determine basal blood glucose levels. The animals, randomly divided into two groups (N ≈ 10-11 per group), were treated daily with vehicle (1% hydroxyethyl cellulose, Sigma-Aldrich) or with tetrahydroquinoline **5a** at 10 mg/kg/day, for 15 days orally. After 15 days of treatment, and 6 h of fasting, the mice were anesthetized by inhalation of isoflurane and blood samples were collected in tubes with heparin or EDTA by intracardiac puncture. Plasma samples were obtained and stored at -80°C. Liver and epididymal white adipose tissue were removed and part of both organs were quickly frozen in liquid N₂ for qPCR studies.

### Biochemical analysis

Plasma levels of total cholesterol and HDL-cholesterol (HDL-c) were determined by enzymatic processes using commercial kits: LabAssay^{™} Cholesterol (Wako Pure Chemicals Industries, Cape Charles, VA) and Mouse HDL-Cholesterol Assay Kit (Crystal Chem, Elk Grove Village, IL), respectively. Non-HDL cholesterol levels were calculated (total cholesterol - HDL-c). Fasting blood glucose levels were measured using a glucometer (Contour Next, Bayer HealthCare Pharmaceuticals LLC, Berlin, Germany), while insulin levels were determined using the commercial Mouse Insulin ELISA kit (Mercodia, Uppsala, Sweden). The HOMA-IR index was calculated using the following formula: fasting blood glucose (mmol/L) * fasting insulinemia (µU/mL) / 22.5.

### Gene expression studies

Gene expression analysis was carried out by real-time qPCR. To this end, total RNA was extracted from liver and epididymal white adipose tissue biopsies using TRIzol reagent (Invitrogen, Carlsbad, CA, USA). The isolated RNA was retro-transcribed using the Maxima First-Strand kit and amplified with a Luminaris Color qPCR MasterMIX (Fermentas, Whaltham, MA, USA) in a 7900 FastSystem thermal cycler. The mRNA levels were normalized to the expression of the constitutive gene cyclophilin.

### Molecular modelling

Molecular modelling was performed using two combined techniques (docking calculations and molecular dynamics simulations). First, docking calculations were performed to localize the different ligands in the binding pockets of different molecular targets using the Autodock 4.2 program. The starting structures were used for molecular dynamics simulations based on the results obtained in the docking study. The 3D crystal structures of PPARγ in complex with rosiglitazone (PDB code:4eMA), PPARα in complex with WY-14643 (PDB code:4BcR), and PPARδ in complex with GW501516 (PDB code:5U46) were used for molecular dynamics simulations. The missing loop (261-275) in PPARγ was modeled based on a previously reported 3D structure (PPARγ1PRG model) (Nolte et al. (1998)). The geometries of the complexes obtained from docking were soaked in boxes of explicit water using the TIP3P model and subjected to molecular dynamics simulation. Molecular dynamics simulations were performed using the Amber 22 software package. The geometry of the system went through a two-step energy minimization process: in the first step, the backbone atoms of the complex were constrained with 10.0 kcal/(mol Å2) force constants; in the second step, all solute and solvent atoms were allowed to move with no constraint to obtain the final relaxed geometry. The non-bonded interaction cutoff was maintained at its default value and the particle mesh Ewald method was used. Simulations were performed for 90 ns for each complex (three runs of 30 ns each). The clustering process was carried out in the following manner: using the 90 ns obtained from the three runs, 15 ns were discarded (the first 5 ns of each individual run). The remaining 75 ns were subjected to a clustering process, from which 10 different families of complexes were obtained. The free energy of decomposition of the residue was calculated using the de mm_pbsa program in Amber22. Each ligand-residue pair includes four energy terms: the van der Waals contribution (ΔEvdw), electrostatic contribution (ΔEele), polar desolvation term (ΔGGB), and nonpolar desolvation term (ΔGSA), which are summarized in the following equation: ΔGligand residue = ΔEvdW + ΔEele + ΔGGB + ΔGSA. Molecular dynamics trajectories and explicit water molecules were removed from the snapshots.

### Statistical analysis

All data are presented as the mean ± SD or ± SEM. Differences between multiple groups were analyzed by one-way analysis of variance (ANOVA), followed by Tukey's or Dunnett's multiple comparisons test (GraphPad Prism 9). Differences with a p-value < 0.05 were considered statistically different.

### Example 2. Synthesis of compounds of Formula I and II

### Materials and methods

### Synthesis of 5-(benzyloxy)-2-nitrobenzaldehyde

A mixture of 5-hydroxy-2-nitrobenzaldehyde (1 g, 5.9 mmol) and K₂CO₃ (0.82 g, 6.51 mmol) was dissolved in dry DMF (12 mL) under N₂, followed by addition of benzyl chloride (1.51 g, 11.9 mmol). The mixture was refluxed for 4 h. The reaction mixture was extracted with CH₂Cl₂ (3 × 10 mL), and the organic layers were combined, washed with brine (1 × 10 mL) and H₂O (2 × 10 mL), dried over anhydrous Na₂SO₄, filtered and then evaporated under reduced pressure. The residue was purified by silica gel column chromatography (hexane/EtOAc, 9:1) to obtain 1.15 g of 5-(benzyloxy)-2-nitrobenzaldehyde as yellow crystals (75% yield). ¹H NMR (300 MHz, CDCl₃): δ 10.46 (s, CHO, 1H), 8.15 (d, *J* = 9.0 Hz, 1H, H-3), 7.43-7.36 (m, 6H, Ph-H, H-6), 7.21 (dd, *J* = 9.0, 2.9 Hz, 1H, H-4), 5.20 (s, 2H, OC*H*₂Ph). ¹³C NMR (75 MHz, CDCl₃): δ 188.5 (CHO), 163.1 (C-5), 142.4 (C-2), 135.0 (C-1'), 134.3 (C-1), 128.9, 128.7 and 127.6 (6C, Ph), 127.3 (CH-3), 119.3 (CH-4), 114.3 (CH-6), 71.1 (OCH₂Ph). MS (ESI) *m*/*z* 256 [M-H]⁺.

### General procedure for the synthesis of quinolines 1a and 1b

A mixture of 5-(benzyloxy)-2-nitrobenzaldehyde (5.88 mmol), Fe (26.5 mmol), and 0.1 N HCl (3.2 mL) was dissolved in absolute ethanol (25 mL) under N₂, refluxed for 30 min, and monitored by TLC. After the reaction was completed, iron solids were removed by centrifugation at 5000 RPM for 5 min. Then, pyrrolidine (1.5 mL, 17.5 mmol) and ethyl levulinate (1.6 mL, 11.6 mmol) were added dropwise, and the reaction mixture was refluxed for additional 4 h. The reaction mixture was extracted in CH₂Cl₂ (3 × 20), washed with brine (3 × 15 mL) and H₂O (3 × 15 mL), dried over anhydrous Na₂SO₄, filtered, and evaporated to dryness. Crude mixture of quinoline isomers 2- and 3- substituted. The residue was purified using silica gel column chromatography (hexane/EtOAc, 8:2).

*6-(Benzyloxy)-2-(ethylpropanoate)-quinoline* (**1a**): 0.96 g, yellow crystals (50% yield). ¹H NMR (300 MHz, CDCl₃): δ 7.80-7.76 (m, 2H, H-4, H-8), 7.35-7.19 (m, 6H, Ph-H, H-7), 7.12 (d, *J* = 7.1 Hz, 1H, H-3), 6.98 (d, *J* = 2.8 Hz, 1H, H-5), 5.02 (s, 2H, OC*H*₂Ph), 4.0 (q, *J* = 7.1 Hz, 2H, OC*H*₂CH₃), 3.12 (t, *J* = 7.5 Hz, 2H, CH₂-1'), 2.74 (t, *J* = 7.5 Hz, 2H, CH₂-2'), 1.09 (t, *J* = 7.1 Hz, 3H, OCH₂C*H*₃). ¹³C NMR (75 MHz, CDCl₃): δ 173.2 (CO₂CH₂CH₃), 158.1 (C-6), 156.4 (C-2), 144.0 (C-8a), 136.6 (C-1"), 135.2 (CH-4), 130.4 (CH-8), 128.7 (2C, CH-3", CH-5"), 128.1 (CH-4"), 127.7 (C-4a), 127.6 (2C, CH-2", CH-6"), 122.3 (CH-7), 121.8 (C-3), 106.6 (CH-5), 70.3 (O*C*H₂Ph), 60.4 (O*C*H₂CH₃), 33.5 and 33.4 (2C, CH₂-1', CH₂-2'), 12.3 (OCH₂*C*H₃). MS (ESI) *m*/*z* 336 [M+H]⁺.

*6-(Benzyloxy)-2-(methyl)-3-(ethylethanoate)-quinoline* (**1b**): 0.71 g, yellow crystals (36% yield). ¹H NMR (300 MHz, CDCl₃): δ 7.96 (d, *J* = 9.1 Hz, 1H, H-8), 7.85 (s, 1H, H-4), 7.49-7.35 (m, 6H, Ph-H, H-7), 7.09 (d, *J* = 3.0 Hz, 1H, H-5), 5.16 (s, 2H, OC*H*₂Ph), 4.18 (q, *J* = 7.1 Hz, 2H, OC*H*₂CH₃), 3.76 (s, 2H, CH₂-1'), 2.70 (s, 3H, CH₃-2), 1.26 (t, *J* = 7.1 Hz, 3H, OCH₂C*H*₃). ¹³C NMR (75 MHz, CDCl₃): δ 170.8 (CO₂CH₂CH₃), 156.5 (C-6), 155.9 (C-2), 143.2 (C-8a), 136.6 (C-1"), 135.8 (CH-4), 130.0 (CH-8), 128.7 (2C, CH-3", CH-5"), 128.2 (CH-4"), 128.0 (C-4a), 127.9 (2C, CH-2", CH-6"), 127.0 (C-3), 122.2 (CH-7), 106.3 (CH-5), 70.3 (O*C*H₂Ph), 61.2 (O*C*H₂CH₃), 39.1 (CH₂-1'), 23.2 (CH₃-2), 14.2 (OCH₂*C*H₃). MS (ESI) *m*/*z* 336 [M+H]⁺.

### General procedure for the synthesis 1,2,3,4-tetrahydroquinolines 2a and 2b

A solution quinoline **1a** (4.77 mmol) or **1b** (4.47 mmol) in dry methanol (40 mL) was treated with sodium cyanoborohydride (38.16 mmol) and boron trifluoride diethyl etherate (38.16 mmol) and refluxed for 4 h under N₂. Then, the reaction mixture was cooled, treated with 15% aqueous NH₃ and extracted with EtOAc (3x 10 mL). The organic layers were combined and washed with H₂O (3 × 10 mL) and brine (3 × 10 mL), dried over anhydrous Na₂SO₄, filtered and dried under vacuum. The crude was subjected to purification by silica gel column chromatography (hexane/EtOAc, 8:2) to afford tetrahydroquinolines **2a** or **2b.**

*6-(Benzyloxy)-2-(ethylpropanoate)-1,2,3,4-tetrahydroquinoline* (**2a**): 1.05 g, yellow oil (65 % yield). ¹H NMR (300 MHz, CDCl₃): δ 7.47-7.29 (m, 5H, Ph-H), 6.72-6.62 (m, 2H, H-5, H-7), 6.45 (d, *J* = 8.7 Hz, 1H, H-8), 4.97 (s, 2H, OC*H*₂Ph), 4.15 (q, *J* = 7.1 Hz, 2H, OC*H*₂CH₃), 3.30-3.22 (m, 1H, CH-2), 2.82-2.67 (m, 2H, CH₂-4), 2.47-2.41 (m, 2H, CH₂-2'), 1.99-1.80 (m, 3H, CH₂-3, CH₂ₐ-1'), 1.68-1.61 (m, 1H, CH_{2b}-1'), 1.27 (t, *J* = 7.1 Hz, 3H, OCH₂C*H*₃). ¹³C NMR (75 MHz, CDCl₃): δ 173.6 (CO₂CH₂CH₃), 151.2 (C-6), 138.7 (C-8a), 137.7 (C-1"), 128.5 (2C, CH-3", CH-5"), 127.7 (CH-4"), 127.5 (2C, CH-2", CH-6"), 122.5 (C-4a), 115.9 (CH-5), 115.4 (CH-7), 114.1 (CH-8), 70.8 (O*C*H₂Ph), 60.5 (O*C*H₂CH₃), 51.1 (CH-2), 31.3 (CH₂-2'), 30.5 (CH₂-1'), 27.6 (CH₂-3), 26.4 (CH₂-4), 14.3 (OCH₂*C*H₃). MS (ESI) *m*/*z* 340 [M+H]⁺.

*6-(Benzyloxy)-3-(ethylethanoate)-2-(methyl)-1,2,3,4-tetrahydroquinoline* (**2b**): 0.91 g, yellow oil (60% yield). ¹H NMR (300 MHz, CDCl3): δ 7.43-7.30 (m, 5H, Ph-H), 6.70-6.63 (m, 2H, H-5, H-7), 6.45 (d, *J* = 8.9 Hz, 1H, H-8), 4.99 (s, 2H, OC*H*₂Ph), 4.13 (q, *J* = 7.1 Hz, 2H, OC*H*₂CH₃), 3.53-3.46 (m, 1H, CH-2), 2.99-2.91 (m, 2H, CH₂ₐ-4), 2.61-2.39 (m, 3H, CH_{2b}-4, CH₂ₐ-1', CH-3), 2.24-2.14 (m, 1H, CH_{2b}-1'), 1.25 (t, *J* = 7.1 Hz, 3H, OCH₂C*H*₃), 1.14 (d, *J* = 6.5 Hz, 3H, CH₃-2). ¹³C NMR (75 MHz, CDCl₃): δ = 173.4 (CO₂CH₂CH₃), 155.6 (C-6), 137.6 (2C, C-8a, C-1"), 128.5 (2C, CH-3", CH-5"), 127.8 (CH-4"), 127.5 (2C, CH-2", CH-6"), 120.3 (C-4a), 116.4 (CH-5), 115.8 (CH-7), 114.2 (CH-8), 70.7 (O*C*H₂Ph), 49.6 (O*C*H₂CH₃), 49.6 (CH-2), 33.7 (CH₂-1'), 32.9 (CH-3), 32.1 (CH₂-4), 17.8 (CH₃-2), 14.3 (OCH₂*C*H₃). MS (ESI) *m*/*z* 340 [M+H]⁺.

### General procedure for the synthesis of N-methyl 1,2,3,4-tetrahydroquinolines 3a and 3b

A mixture of tetrahydroquinoline **2a** (1.47 mmol) or **2b** (1.47 mmol), and iodomethane (7.2 mmol) in anhydrous DMF (15 mL) was refluxed at 140 °C for 15 min. The reaction mixture was then cooled, diluted with distilled water (10 mL), and extracted with CH₂Cl₂ (3 × 10 mL). The organic layers were combined, washed with H₂O (3 × 10 mL), brine (3 × 10 mL), dried over anhydrous Na₂SO₄, filtered and evaporated to dryness. The residue was purified by silica gel column chromatography (hexane/EtOAc, 9:1) to afford N-methyl tetrahydroquinolines **3a** or **3b**.

*6-(Benzyloxy)-2-(ethylpropanoate)-N-(methyl)-1,2,3,4-tetrahydroquinoline* (**3a**): 413.5 mg, brown oil (80% yield). ¹H NMR (300 MHz, CDCl₃): δ 7.44-7.27 (m, 5H, Ph-H), 6.79 (dd, 1H, *J* = 8.8, 3.0, H-7), 6.73 (d, *J* = 3.0, H-5), 6.52 (d, *J* = 8.8, H-8), 4.98 (s, 2H, OC*H*₂Ph), 4.13 (q, *J* = 7.1 Hz, 2H, OC*H*₂CH₃), 3.25-3.12 (m, 1H, CH-2), 2.91 (s, 3H, N-CH₃), 2.77-2.63 (m, 2H, CH₂-4), 2.38-2.33 (m, 2H, CH₂-2'), 1.92-1.55 (m, 4H, CH₂-3, CH₂-1'), 1.25 (t, *J* = 7.1 Hz, 3H, OCH₂C*H*₃). ¹³C NMR (75 MHz, CDCl₃): δ 173.5 (CO₂CH₂CH₃), 150.1 (C-6), 140.2 (C-8a), 137.8 (C-1"), 128.5 (2C, CH-3", CH-5"), 127.7 (CH-4"), 127.5 (2C, CH-2", CH-6"), 123.4 (C-4a), 116.1 (CH-5), 113.6 (CH-7), 112.3 (CH-8), 70.9 (O*C*H₂Ph), 60.5 (O*C*H₂CH₃), 58.0 (CH-2), 38.8 (N-CH₃), 30.9 (CH₂-2'), 26.3 (CH₂-1'), 24.6 (CH₂-3), 23.9 (CH₂-4), 14.3 (OCH₂*C*H₃). MS (ESI) *m*/*z* 354 [M+H]⁺.

*6-(Benzyloxy)-3-(ethylethanoate)-2-(methyl)-N-(methyl)-1,2,3,4-tetrahydroquinoline* (**3b**): 388.7 mg, brown oil (75% yield). ¹H NMR (300 MHz, CDCl₃): δ 7.46-7.29 (m, 5H, Ph-H), 6.79 (dd, *J* = 8.7, 3.1 Hz, 1H, H-7), 6.72 (d, *J* = 3.1 Hz, 1H, H-5), 6.47 (d, *J* = 8.7 Hz, 1H, H-8), 4.99 (s, 2H, OC*H*₂Ph), 4.14 (q, *J* = 7.1 Hz, 2H, OC*H*₂CH₃), 3.20-3.17 (m, 1H, CH-2), 3.11-3.04 (m, 1H, CH₂ₐ-4), 2.87 (s, 3H, N-CH₃), 2.50-2.48 (m, 1H, CH_{2b}-4), 2.43-2.25 (m, 3H, CH₂-1', CH-3), 1.25 (t, *J* = 7.1 Hz, 3H, OCH₂C*H*₃), 1.09 (d, *J* = 6.5 Hz, 3H, CH₃-2). ¹³C NMR (75 MHz, CDCl₃): δ 173.4 (CO₂CH₂CH₃), 150.1 (C-6), 139.0 (C-8a), 137.8 (C-1"), 128.5 (2C, CH-3", CH-5"), 127.7 (CH-4"), 127.5 (2C, CH-2", CH-6"), 120.3 (C-4a), 116.9 (CH-5), 113.7 (CH-7), 111.3 (CH-8), 70.8 (O*C*H₂Ph), 60.2 (O*C*H₂CH₃), 58.1 (CH-2), 38.3 (CH₂-1'), 37.7 (N-CH₃), 33.5 (CH-3), 28.6 (CH₂-4), 16.7 (OCH₂*C*H₃), 14.3 (CH₃-2). MS (ESI) *m*/*z* 353 [M]⁺.

### General procedure for the synthesis of aldehydes 4a, 4b, 7a and 7b

Solutions of quinoline or tetrahydroquinolines esters **1a**, **1b**, **3a** or **3b** (0.60 mmol) in dry CH₂Cl₂ (5 mL) were treated with 1 M DIBAL-H (1.2 mmol) at -78 °C under N₂ and stirred for 15 min. Then, methanol (1 mL) was added dropwise at -78 °C to quench the reaction, and the mixture was stirred for 15 min, followed by saturated NH₄Cl (1 mL) and stirred for 15 min to room temperature. The reaction mixture was extracted with EtOAc (3 × 10 mL) and the organic layers were combined and washed with brine (2 × 10 mL) and H₂O (2 × 10 mL), dried over anhydrous Na₂SO₄, filtered and evaporated to dryness. The residues were purified by silica gel column chromatography (hexane/EtOAc, 7:3) to obtain tetrahydroquinoline aldehydes **4a** and **4b**, and quinoline aldehydes **7a** and **7b**.

*6-(Benzyloxy)-N-(methyl)-2-(propanal)-1,2,3,4-tetrahydroquinoline* (**4a**): 130 mg, light-yellow oil (70% yield). ¹H NMR (300 MHz, CDCl₃): δ 9.70 (t, *J* = 2.2 Hz, 1H, CHO), 7.46-7.32 (m, 5H, Ph-H), 6.80 (dd, *J* = 8.8, 2.9 Hz, 1H, H-7), 6.72 (d, *J* = 2.9 Hz, 1H, H-5), 6.57 (d, *J* = 8.8 Hz, 1H, H-8), 5.01 (s, 2H, OC*H*₂Ph), 3.26-3.11 (m, 1H, CH-2), 2.90 (s, 3H, N-CH₃), 2.85-2.62 (m, 2H, CH₂-4), 2.58-2.34 (m, 2H, CH₂-2'), 2.08-1.62 (m, 4H, CH₂-3, CH₂-1'). ¹³C NMR (75 MHz, CDCl₃): δ 201.8 (CHO), 150.6 (C-6), 140.1 (C-8a), 137.8 (C-1"), 128.5 (2C, CH-3", CH-5"), 127.8 (CH-4"), 127.5 (2C, CH-2", CH-6"), 123.8 (C-4a), 115.9 (CH-5), 113.8 (2C, C-7, C-8), 70.8 (O*C*H₂Ph), 58.2 (CH-2), 40.7 (CH₂-2'), 39.7 (N-CH₃), 24.3, 24.1 and 23.9 (3C, CH₂-3, CH₂-1', CH₂-4). MS (ESI) *m*/*z* 310 [M+H]⁺.

*6-(Benzyloxy)-3-(ethanal)-2-(methyl)-N-(methyl)-1,2,3,4-tetrahydroquinoline* (**4b**): 148.5 mg, light-yellow oil (80% yield). ¹H NMR (300 MHz, CDCl₃): δ 9.76 (t, *J* = 1.3 Hz, 1H, CHO), 7.44-7.29 (m, 5H, Ph-H), 6.78 (dd, *J* = 8.8, 3.0 Hz, 1H, H-7), 6.69 (d, *J* = 3.0 Hz, 1H, H-5), 6.47 (d, *J* = 8.8 Hz, 1H, H-8), 4.97 (s, 2H, OC*H*₂Ph), 3.15-3.06 (m, 2H, CH-2, CH₂ₐ-4), 2.85 (s, 3H, N-CH₃), 2.53-2.37 (m, 4H, CH₂-1', CH_{2b}-4, CH-3), 1.08 (d, *J* = 6.4 Hz, CH₃-2). ¹³C NMR (75 MHz, CDCl₃): δ. 202.1 (CHO), 150.4 (C-6), 138.9 (C-8a), 137.7 (C-1"), 128.5 (2C, CH-3", CH-5"), 127.8 (CH-4"), 127.6 (2C, CH-2", CH-6"), 120.3 (C-4a), 116.8 (CH-5), 113.8 (CH-7), 111.6 (CH-8), 70.8 (O*C*H₂Ph), 58.1 (CH-2), 48.1 (CH₂-1'), 37.7 (N-CH₃), 31.1 (CH-3), 28.6 (CH₂-4), 16.4 (CH₃-2). MS (ESI) *m*/*z* 310 [M+H]⁺.

*6-(Benzyloxy)-2-(propanal)-quinoline* (**7a**): 87.4 mg, light-yellow oil (50% yield). ¹H NMR (300 MHz, CDCl₃): δ 9.78 (t, *J* = 1.3 Hz, 1H, CHO), 7.80-7.75 (m, 2H, H-4, H-8), 7.35-7.18 (m, 6H, Ph-H, H-7), 7.11 (d, *J* = 9.1 Hz, 1H, H-3), 6.97 (d, *J* = 2.8 Hz, 1H, H-5), 5.01 (s, 2H, OC*H*₂Ph), 3.13 (t, *J* = 7.0 Hz, 2H, CH₂-1'), 2.88 (td, *J* = 7.0, 1.3 Hz, 2H CH₂-2'). ¹³C NMR (75 MHz, CDCl₃): δ: 201.8 (CHO), 157.7 (C-6), 156.5 (C-2), 144.0 (C-8a), 136.6 (C-1"), 135.3 (CH-4), 130.4 (CH-8), 128.7 (2C, CH-3", CH-5"), 128.2 (CH-4"), 127.7 (C-4a), 127.6 (2C, CH-2", CH-6"), 122.4 (CH-3), 121.7 (CH-7), 106.6 (CH-5), 70.3 (O*C*H₂Ph), 42.4 (CH₂-2'), 30.9 (CH₂-1'). MS (ESI) *m*/*z* 290 [M-H]⁺.

*6-(Benzyloxy)-3-(ethanal)-2-(methyl)-quinoline* (**7b**): 131.3 mg, light-yellow oil (75% yield). ¹H NMR (300 MHz, CDCl₃): δ 9.82 (t, *J* = 1.8 Hz, 1H, CHO), 7.97 (d, *J* = 9.2 Hz, 1H, H-8), 7.82 (s, 1H, H-4), 7.49-7.34 (m, 6H, Ph-H, H-7), 7.10 (d, *J* = 2.8 Hz, 1H, H-5), 5.17 (s, 2H, OC*H*₂Ph), 3.88 (d, *J* = 1.8 Hz, 2H, CH₂-1'), 2.65 (s, 3H, CH₃-2). ¹³C NMR (75 MHz, CDCl₃): δ 197.9 (CHO), 156.8 (C-6), 155.4 (C-2), 142.6 (C-8a), 136.8 (CH-4), 136.4 (C-1"), 129.4 (CH-8), 128.7 (2C, CH-3", CH-5"), 128.2 (CH-4"), 128.0 (C-4a), 127.5 (2C, CH-2", CH-6"), 125.0 (C-3), 122.8 (CH-7), 106.1 (CH-5), 70.2 (O*C*H₂Ph), 48.0 (CH₂-1'), 22.9 (CH₃-2). MS (ESI) *m*/*z* 290 [M-H]⁺.

### General procedure for the synthesis of 2- and 3-prenylated of tetrahydroquinoline and quinoline esters 5a, 5b, 8a and 8b

A mixture of aldehydes **4a**, **4b**, **7a** or **7b** (0.2 mmol) and 0.5 M isopropenylmagnesium bromide (0.6 mmol) in THF (10 mL) at -78 °C was stirred for 1 h under N₂. The reaction mixture was quenched by adding saturated aqueous NH₄Cl (1 mL) dropwise, and stirred for additional 15 min at room temperature. Then, H₂O (5 mL) was then added and the mixture was extracted with EtOAc (3 × 10 mL). The combined organic layers were washed with H₂O (3 × 10 mL), brine (3 × 10 mL), dried over anhydrous Na₂SO₄, filtered and evaporated under reduced pressure. The residue, without further purification, was treated with triethyl orthoacetate (10 mL) and catalytic amounts of isobutyric acid (3 drops). The mixture was refluxed at 140 °C for 2 h, and after concentrated under reduced pressure to remove excess triethyl orthoacetate. The residue was diluted with CH₂Cl₂ (10 mL), washed with H₂O (3 × 10 mL), brine (3 × 10 mL), dried over anhydrous Na₂SO₄, filtered and concentrated under vacuum. The crude was purified by silica gel column chromatography (hexane/EtOAc, 80:20) to afford compounds **5a**, **5b**, **8a** or **8b**.

*Ethyl (E)-7-(6-(benzyloxy)-1-methyl-1,2,3,4-tetrahydroquinolin-2-yl)-4-methylhept-4-enoate* (**5a**): 37.9 mg, amber oil (45% yield). ¹H NMR (300 MHz, CDCl₃): δ 7.44-7.30 (m, 5H, Ph-H), 6.75 (dd, *J* = 8.8, 3.0 Hz, 1H, H-7), 6.70 (d, *J* = 3.0 Hz, 1H, H-5), 6.47 (d, *J* = 8.8 Hz, 1H, H-8), 5.19-5.14 (m, 1H, CH-3'), 4.98 (s, 2H, OC*H*₂Ph), 4.12 (q, *J* = 7.1 Hz, 2H, OC*H*₂CH₃), 3.18-3.16 (m, 1H, CH-2), 2.88 (s, 3H, N-CH₃), 2.85-2.56 (m, 2H, CH₂-4), 2.45-2.24 (m, 4H, CH₂-6', CH₂-5'), 2.13-1.87 (m, 4H, CH₂₋2', CH₂-3), 1.62 (s, 3H, CH₃-4'), 1.63-1.61 (m, 1H, CH₂ₐ-1'), 1.43-1.39 (m, 1H, CH_{2b}-1'), 1.24 (t, *J* = 7.1 Hz, 3H, OCH₂C*H*₃). ¹³C NMR (75 MHz, CDCl₃): δ 173.3 (CO₂CH₂CH₃), 149.8 (C-6), 140.3 (C-8a), 137.8 (C-1"), 133.7 (C-4'), 128.4 (2C, CH-3", CH-5"), 127.6 (CH-4"), 127.4 (2C, CH-2", CH-6"), 124.7 (CH-3'), 123.3 (C-4a), 116.0 (CH-5), 113.4 (CH-7), 111.7 (CH-8), 70.8 (O*C*H₂Ph), 60.2 (O*C*H₂CH₃), 58.2 (CH-2), 38.3 (N-CH₃), 34.6 (CH₂-5'), 33.1 (CH₂-6'), 30.4 (CH₂-1'), 24.6 and 24.4 (2C, CH₂-3, CH₂-2'), 23.9 (CH₂-4) 15.9 (CH₃-4'), 14.2 (OCH₂*C*H₃). HRMS (ESI⁺) *m*/*z*: calcd. for C₂₇H₃₆NO₃ [M+H]⁺ 422.2690, found 422.2691.

*Ethyl (E)-6-(6-(benzyloxy)-1,2-dimethyl-1,2,3,4-tetrahydroquinolin-3-yl)-4-methylhex-4-enoate* (**5b**): 32.0 mg, amber oil (38% yield). ¹H NMR (300 MHz, CDCl₃): δ 7.41-7.25 (m, 5H, Ph-H), 6.71 (dd, *J* = 8.7, 3.0 Hz, 1H, H-7), 6.64 (d, *J* = 3.0 Hz, 1H, H-5), 6.39 (d, *J* = 8.7 Hz, 1H, H-8), 5.14 (m, 1H, CH-2'), 4.93 (s, 2H, OC*H*₂Ph), 4.08 (q, *J* = 7.1 Hz, 2H, OC*H*₂CH₃), 3.06-3.01 (m, 1H, CH-2), 2.93-2.86 (m, 1H, CH₂ₐ-4), 2.79 (s, 3H, N-CH₃), 2.40-2.23 (m, 5H, CH_{2b}-4, CH₂-5', CH₂-4'), 2.01-1.79 (m, 2H, CH₂-1'), 1.65-1.60 (m, 1H, CH-3), 1.45 (s, 3H, CH₃-3'), 1.21 (t, *J* = 7.2 Hz, 3H, OCH₂C*H*₃), 0.99 (d, *J* = 6.5 Hz, 3H, CH₃-2). ¹³C NMR (75 MHz, CDCl₃+ 1 drop CD₃OD): δ 173.8 (CO₂CH₂CH₃), 149.8 (C-6), 139.4 (C-8a), 137.8 (C-1"), 134.8 (C-3'), 128.4 (2C, CH-3", CH-5"), 127.7 (CH-4"), 127.5 (2C, CH-2", CH-6"), 123.8 (CH-2'), 121.5 (C-4a), 116.8 (CH-5), 113.4 (CH-7), 110.8 (CH-8), 70.9 (O*C*H₂Ph), 60.4 (O*C*H₂CH₃), 57.3 (CH-2), 37.6 (CH-3), 34.8 (N-CH₃), 33.3 (2C, CH₂-4', CH₂-5'), 31.8 (CH₂-1'), 28.7 (CH-4), 17.1 (CH₃-2), 15.9 (CH₃-3'), 14.2 (OCH₂*C*H₃). HRMS (ESI⁺) *m*/*z*: calcd. for C₂₇H₃₆NO₃ [M+H]⁺ 422.2690, found 422.2686.

*Ethyl (E)-7-(6-(benzyloxy)quinolin-2-yl)-4-methylhept-4-enoate* (**8a**): 36.3 mg, amber oil (45% yield). ¹H NMR (300 MHz, CDCl₃): δ 8.01 (d, *J* = 9.3 Hz, 1H, H-8), 8.00 (d, *J* = 8.3 Hz, 1H, H-4), 7.50-7.31 (m, 6H, Ph-H, H-7), 7.24 (d, *J* = 8.3 Hz, 1H, H-3), 7.14 (d, *J* = 2.8 Hz, 1H, H-5), 5.29-5.22 (m, 1H, CH-3'), 5.17 (s, 2H, OC*H*₂Ph), 4.09 (q, *J* = 7.2 Hz, 2H, OC*H*₂CH₃), 3.00-2.95 (m, 2H, CH₂-1'), 2.54-2.49 (m, 2H, CH₂-2'), 2.39-2.26 (m, 4H, CH₂-5', CH₂-6'), 1.55 (s, 3H, CH₃-3') 1.22 (t, *J* = 7.1 Hz, 3H, OCH₂C*H*₃). ¹³C NMR (75 MHz, CDCl₃): δ 173.5 (CO₂CH₂CH₃), 159.7 (C-6), 156.5 (C-2), 136.6 (C-8a), 135.5 (CH-4), 134.6 (2C, C-1", C-4'), 129.9 (CH-8), 128.7 (2C, CH-3", CH-5"), 128.2 (CH-4"), 127.6 (C-4a) 127.5 (2C, CH-2", CH-6"), 124.0 (CH-3'), 122.5 (CH-7), 121.8 (CH-3), 106.6 (CH-5), 70.2 (O*C*H₂Ph), 60.3 (O*C*H₂CH₃), 38.6 (CH₂-1'), 34.6 and 33.2 (2C, CH₂-5', CH₂-6'), 28.4 (CH₂-2'), 16.0 (CH₃-4'), 14.3 (OCH₂*C*H₃). HRMS (ESI⁺) *m*/*z*: calcd. for C₂₆H₃₀NO₃ [M+H]⁺ 404.2220, found 404.2221.

*Ethyl (E)-6-(6-(benzyloxy)-2-methylquinolin-3-yl)-4-methylhex-4-enoate* (**8b**): 37.9 mg, amber oil (47 % yield). ¹H NMR (300 MHz, CDCl₃): δ 7.90 (d, *J* = 9.1 Hz, 1H, H-8), 7.70 (s, 1H, H-4), 7.54-7.30 (m, 6H, Ph-H, H-7), 7.10 (d, *J* = 2.8 Hz, 1H, H-5), 5.38-5.32 (m, 1H, CH-2'), 5.16 (s, 2H, OC*H*₂Ph), 4.10 (q, *J* = 7.2 Hz, 2H, OC*H*₂CH₃), 3.48-3.37 (m, 2H, CH₂-1'), 2.65 (s, 3H, CH₃-2), 2.51-2.35 (m, 4H, CH₂-4', CH₂-5'), 1.76 (s, 3H, CH₃-3'), 1.19 (t, *J* = 7.1 Hz, 3H, OCH₂C*H*₃). ¹³C NMR (75 MHz, CDCl₃ + 1 drop CD₃OD): δ 173.8 (CO₂CH₂CH₃), 149.8 (2C, C-6, C-2), 139.5 (C-8a), 137.8 (2C, C-1", C-4a), 134.8 (C-3'), 133.6 (C-3), 128.4 (2C, CH-3", CH-5"), 127.7 (CH-4"), 127.5 (2C, CH-2", CH-6"), 123.8 (CH-8), 121.45 (CH-4) 116.8 (CH-2'), 113.4 (CH-7), 110.8 (CH-5), 70.9 (O*C*H₂Ph), 60.4 (O*C*H₂CH₃), 37.6 (CH₂-4'), 34.8 (CH₂-5'), 33.3 (CH₂-1'), 17.1 (CH₃-2), 15.9 (CH₃-3'), 14.2 (OCH₂*C*H₃). HRMS (ESI⁺) *m*/*z:* calcd. for C₂₆H₃₀NO₃ [M+H]⁺ 404.2220, found 404.2220.

### General procedure for the synthesis of 2- and 3-prenylated tetrahydroquinoline and quinoline 6a, 6b, 9a and 9b carboxylic acids

Tetrahydroquinoline **5a** or **5b** and quinoline esters **8a** or **8b** (0.05 mmol) were treated with aqueous 20% KOH (200 µL) in ethanol (10 mL) at reflux for 4 h under N₂. The reaction mixture was extracted with EtOAc (3 × 10 mL) and the combined organic layers were washed with H₂O (3 × 10 mL) and brine (3 × 10 mL), dried over anhydrous Na₂SO₄, filtered and evaporated to dryness. The residue was purified by silica gel column chromatography (CH₂Cl₂/MeOH, 90:10) to afford tetrahydroquinolines **5a** and **5b** and quinoline carboxylic acids **9a** and **9b**.

*(E)-7-(6-(benzyloxy)-1-methyl-1,2,3,4-tetrahydroquinolin-2-yl)-4-methylhept-4-enoic acid* (**6a**): 17.7 mg, light-yellow oil (90 % yield). ¹H NMR (300 MHz, CDCl₃): δ 7.45-7.31 (m, 5H, Ph-H), 6.74 (dd, *J* = 9.0, 3.0 Hz, 1H, H-7), 6.67 (d, *J* = 3.0 Hz, 1H, H-5), 6.41 (d, *J* = 9.0 Hz, 1H, H-8), 5.20-5.16 (m, 1H, CH-3'), 4.99 (s, 2H, OC*H*₂Ph), 3.20-3.15 (m, 1H, CH-2), 2.88 (s, 3H, N-CH₃), 2.81-2.62 (m, 2H, CH₂-4), 2.47-2.41 (m, 2H, CH₂-6'), 2.33-2.28 (m, 2H, CH₂-5'), 2.05-1.86 (m, 4H, CH₂-2', CH₂-3), 1.63 (s, 3H, CH₃-4'), 1.61-1.38 (m, 2H, CH₂-1'). ¹³C NMR (75 MHz, CDCl₃): δ 177.2 (*C*O₂H), 150.0 (C-6), 140.3 (C-8a), 137.9 (C-1"), 133.7 (C-4'), 128.5 (2C, CH-3", CH-5"), 127.7 (CH-4"), 127.5 (2C, CH-2", CH-6"), 124.9 (CH-3'), 123.1 (C-4a), 116.1 (CH-5), 113.5 (CH-7), 111.9 (CH-8), 70.9 (O*C*H₂Ph), 58.3 (CH-2), 38.4 (N-CH₃), 34.5 (CH₂-5'), 32.9 (CH₂-6'), 30.4 (CH₂-1'), 24.5 (CH₂-3), 24.4 (CH₂-2'), 24.0 (CH₂-4), 16.0 (CH₃-4'). HRMS (ESI⁺) *m*/*z:* calcd. for C₂₅H₃₂NO₃ [M+H]⁺ 394.2377, found 394.2377.

*(E)-6-(6-(benzyloxy)-1,2-dimethyl-1,2,3,4-tetrahydroquinolin-3-yl)-4-methylhex-4-enoic acid* (**6b**): 18.1 mg, light-yellow oil (92 % yield). ¹H NMR (300 MHz, CDCl₃): δ 7.41-7.27 (m, 5H, Ph-H), 6.74 (dd, *J* = 8.6, 1.8 Hz, 1H, H-7), 6.67 (d, *J* = 1.8 Hz, 1H, H-5), 6.41 (d, *J* = 8.6 Hz, 1H, H-8), 5.20-5.16 (m, 1H, CH-2'), 4.95 (s, 2H, OC*H*₂Ph), 3.08-3.06 (m, 1H, CH-2), 3.01-2.85 (m, 1H, CH₂ₐ-4), 2.81 (s, 3H, N-CH₃), 2.45-1.65 (m, 7H, CH_{2b}-4, CH₂-5', CH₂-4', CH₂-1'), 1.48 (s, 3H, CH₃-3'), 1.01 (d, *J* = 6.6 Hz, 3H, CH₃-2). ¹³C NMR (75 MHz, CDCl₃): δ 179.0 (*C*O₂H), 150.0 (C-6), 139.4 (C-8a), 137.8 (C-1"), 134.8 (C-3'), 128.5 (2C, CH-3", CH-5"), 127.8 (CH-4"), 127.5 (2C, CH-2", CH-6"), 123.9 (CH-2'), 121.4 (C-4a), 116.7 (CH-5), 113.5 (CH-7), 110.9 (CH-8), 70.9 (O*C*H₂Ph), 57.7 (CH-2), 37.7 (CH-3), 34.6 (N-CH₃), 32.3 (2C, CH₂-4', CH₂-5'), 31.7 (CH₂-1'), 28.7 (CH-4), 17.0 (CH₃-2), 16.0 (CH₃-3'). HRMS (ESI⁺) *m*/*z:* calcd. for C₂₅H₃₁NO [M]⁺ 394.2377, found 394.2368.

*(E)-7-(6-(benzyloxy) quinolin-2-yl)-4-methylhept-4-enoic acid* (**9a**): 15.0 mg, light-yellow oil (80 % yield). ¹H NMR (300 MHz, CDCl₃): δ 8.71 (d, *J* = 9.2 Hz, 1H, H-8), 8.28 (d, *J* = 8.4 Hz, 1H, H-4), 7.58 (dd, *J* = 9.2, 3.0 Hz, 1H, H-7), 7.63-7.36 (m, 7H, Ph-H, H-5, H-3), 5.23-5.19 (m, 1H, CH-3'), 5.19 (s, 2H, OC*H*₂Ph), 3.35-3.30 (m, 2H, CH₂-1'), 2.63-2.58 (m, 2H, CH₂-2'), 2.38-2.34 (m, 2H, CH₂-5'), 2.25-2.20 (m, 2H, CH₂-6'), 1.45 (s, 3H, CH₃-4'). ¹³C NMR (75 MHz, CDCl₃): δ 175.9 (*C*O₂H), 158.3 (C-6), 157.7 (C-2), 147.7 (C-8a), 141.2 (CH-4), 136.0 (C-1"), 135.6 (C-4'), 128.8 (3C, CH-3", CH-5", C-4a), 128.5 (CH-4"), 127.6 (2C, CH-2", CH-6"), 126.1 (CH-7), 125.3 (CH-8), 122.5 (CH-3'), 122.0 (CH-3), 106.7 (CH-5), 70.7 (O*C*H₂Ph), 34.8 (CH₂-1'), 34.7 (CH₂-6'), 32.9 (CH₂-5'), 27.9 (CH₂-2'), 15.9 (CH₃-4'). HRMS (ESI⁺) *m*/*z*: calcd. for C₂₄H₂₆NO₃ [M+H]⁺ 376.1907, found 376.1909.

*(E)-6-(6-(benzyloxy)-2-methylquinolin-3-yl)-4-methylhex-4-enoic acid* (**9b**): 15.9 mg, light-yellow oil (85 % yield). ¹H NMR (300 MHz, CDCl₃): δ 8.03 (d, *J* = 9.2 Hz, 1H, H-8), 7.79 (s, 1H, H-4), 7.51-7.30 (m, 6H, Ph-H, H-7), 7.07 (d, *J* = 2.7 Hz, 1H, H-5), 5.37-5.32 (m, 1H, CH-2'), 5.10 (s, 2H, OC*H*₂Ph), 3.40 (d, *J* = 7.0 Hz, 2H, CH₂-1'), 2.65 (s, 3H, CH₃-2), 2.55-2.51 (m, 2H, CH₂-4'), 2.48-2.45 (m, 2H, CH₂-5'), 1.76 (s, 3H, CH₃-3'). ¹³C NMR (75 MHz, CDCl₃ + 1 drop CD₃OD): δ 175.7 (CO₂H), 156.6 (C-6), 155.9 (C-2), 136.6 and 136.4 (C-8a, C-4a), 134.8 (2C, C-1", C-3'), 133.8 (2C, CH-4, C-3), 128.6 (2C, CH-3", CH-5"), 128.4 (CH-8), 128.1 (CH-4"), 127.5 (2C, CH-2", CH-6"), 122.1 (CH-7), 121.3 (CH-2'), 106.3 (CH-5), 70.3 (O*C*H₂Ph), 34.5 (CH₂-5'), 32.7 (CH₂-4'), 31.4 (CH₂-1'), 21.7 (CH₃-2), 16.1 (CH₃-3'). HRMS (ESI⁺) *m*/*z*: calcd. for C₂₄H₂₅NO₃ [M]⁺ 376.1907, found 376.1907.

### Results

The synthetic routes to prepare the 2-prenylated (series a) and 3-prenylated (series b) quinolines and tetrahydroquinolines are depicted in Scheme 1. The quinoline nucleus was obtained *via* a Friedländer reaction, which required o-nitrobenzaldehyde being reduced into o-aminobenzaldehyde, followed by the condensation of this intermediate (without further purification) with an enolizable ketone ethyl levulinate. Hence, the nitro group was reduced using iron powder in the presence of aqueous HCl. The best results were obtained after centrifuging the reaction mixture of the reduction of the nitro- to the amine-group to remove iron solids, and by treating the supernatant with ethyl levulinate in the presence of pyrrolidine. Under these conditions, Friedländer condensation allowed us to prepare both 2-substituted quinoline **1a** (series a) and 2-methyl-3-substituted quinoline **1b** (series b) at the 1.4:1 ratio with a good yield. Four tetrahydroquinolines (**5a**, **6a**, **5b**, **6b**) and four quinolines (**8a**, **9a**, **8b**, **9b**) from 2-monosubstituted quinoline **1a** (series a) and 2-methyl-3-substituted quinoline **1b** were prepared by the elongation of the side carbon chain at 2-position or 3-position, respectively. The pyridine rings of **1a** and **1b** were selectively reduced under Lewis acidic conditions using a combination of sodium cyanoborohydride and boron trifluoride diethyl etherate to give tetrahydroquinolines **2a** and **2b**, respectively. Subsequently, the *N*-methylation of **2a** and **2b** allowed *N*-methyl- tetrahydroquinolines **3a** and **3b**, respectively. In a second approach, 2-prenylated tetrahydroquinolines **5a** and quinoline **8a** bearing a seven-carbon chain were obtained, as well as 2-methyl-3-prenylated tetrahydroquinoline **5b** and quinoline **8b** bearing a six-carbon chain. The hydrolysis of esters gave tetrahydroquinolines **6a** and **6b** as well as quinolines **9a** and **9b** for series a and b, respectively. 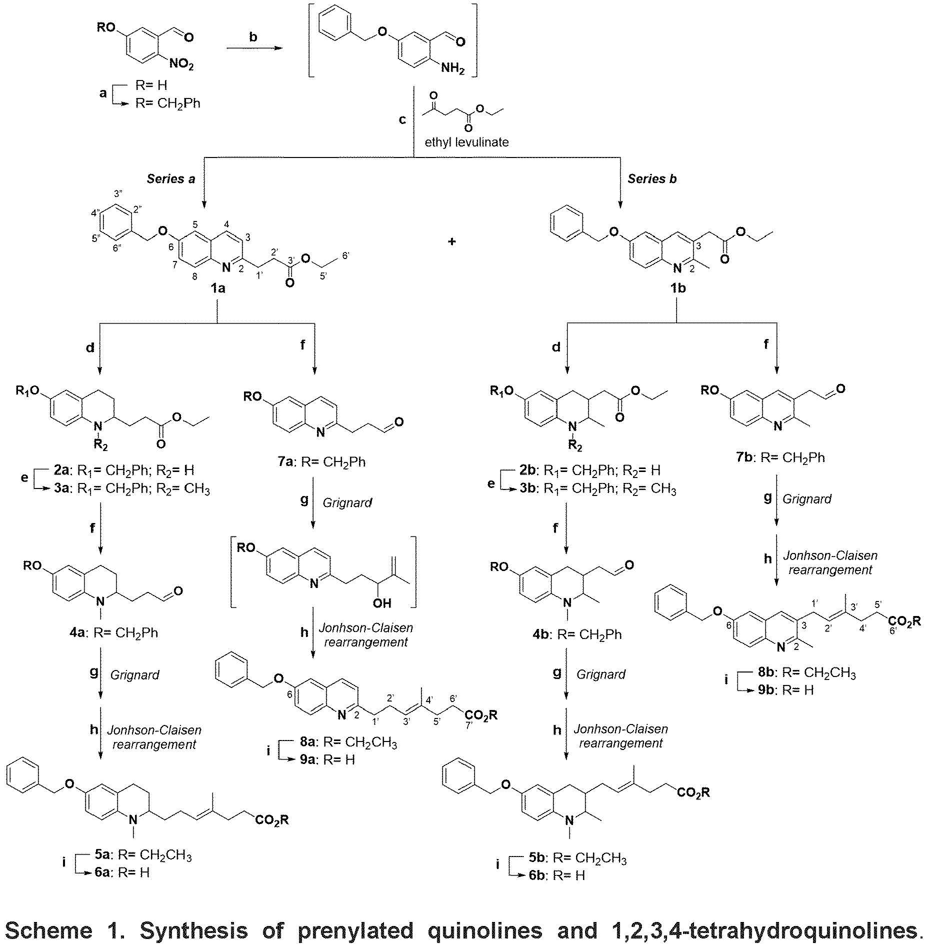 *Reagents and conditions:* (a) K₂CO₃, PhCH₂Cl, DMF, reflux, 4 h (75%); (b) Fe/HCl, EtOH, reflux, 30 min; (c) Pyrrolidine, EtOH, reflux, 4 h (50% for **1a**, 36% for **1b**); (d) NaBH₃CN, BF₃OEt, MeOH, reflux, 3 h (65% for **2a**, 60% for **2b**); (e) CH₂O/HCO₂H, EtOH, 1 h, reflux; NaBH₄, 25 °C, 30 min (80% for **3a**, 75% for **3b**); (f) DIBAL-H, CH₂Cl₂, -78 °C, 15 min (80% for **4a**, 7 % for **4b**, 50% for **7a**, 75% for **7b**); (g) 0.5 M CH₂=C(CH₃)MgBr, THF, -78°C, 1 h; (h) MeC(OEt)₃, isobutyric acid, 140 °C, 2 h (45% for **5a**, 38% for **5b**, 47% for **8a**, 40% for **8b**); (i) 20% KOH, MeOH, reflux, 2 h (90% for **6a**, 92% for **6b**, 80% for **9a**, 85% for **9b**).

### Example 3. PPARα, PPARβ/δ and PPARγ agonist activity of compounds of Formula I and II

Tetrahydroquinolines **5a**, **6a**, **5b**, and **6b**, as well as quinolines **8a**, **9a**, **8b**, and **9b**, were assayed *in vitro* for hPPARa, hPPARβ/δ and hPPARy transactivation activity. Activity was referred to as the maximal activity (Eₘₐₓ) obtained for each tetrahydroquinoline or quinoline at 10 µM expressed as a percentage of maximal activity of the following prior art compounds: WY-14,643 at 10 µM for hPPARa, and rosiglitazone or GW501516 at 1 µM for hPPARy or hPPARβ/δ respectively.

The synthesized quinolines and tetrahydroquinolines have shown pan-PPAR agonist activity (**5a**, **6a**, **8a**) and dual PPARα/γ agonist activity (**9a**, **8b**, **9b**), always with partial activity on the PPARγ isoform. The 3-substituted tetrahydroquinolines (**5b**, **6b**) were selective PPARα, with efficiencies 3-4 times higher than the prior art compound WY-14,643. **Table 2** shows the evaluation of agonist activity in a cell-based transactivation assays for human PPAR/Gal₄ receptors. EC₅₀ values against human PPARα/Gal₄, PPARβ/δ/Gal₄ and PPARγ/Gal₄ receptors.

**Table 2**

| **Compound** | **Efficacy (%) at 10** | | **µmol/L** | **EC₅₀ (µmol/L)** | | |
|---|---|---|---|---|---|---|
| | **PPARα** | **PPARβ/δ** | **PPARγ** | **PPARα** | **PPARβ/δ** | **PPARγ** |
| **5a** | 76 | 60 | 43 | 3.6 | 2.6 | 2.7 |
| **6a** | 144 | 73 | 36 | 2.9 | 4.6 | 3.9 |
| **5b** | 328 | 7 | 15 | 1.9 | 5.8 | 16.9 |
| **6b** | 461 | 35 | 24 | 1.3 | 5.8 | 1.7 |
| **8a** | 210 | 32 | 66 | 2.8 | 2.0 | 1.4 |
| **9a** | 104 | 14 | 39 | 3.6 | 1.1 | 3.3 |
| **8b** | 221 | 2 | 37 | 2.2 | 7.9 | 1.0 |
| **9b** | 218 | 2 | 25 | 1.8 | 5.1 | 0.9 |
| WY-14,643 | 100 | NA | NA | 1.3 | NA | NA |
| GW501516 | NA | 100 | NA | NA | 0.9 | NA |
| Rosiglitazone | NA | NA | 100 | NA | NA | 0.1 |
| *Note.* Efficacy: Emax is the maximal PPAR fold-activation relative to the maximum activation obtained with WY-14,643 (10 µM), GW501516 (1 µM), and rosiglitazone (1 µM), corresponding to 100% activation in the GAL4 chimeric hPPARα, hPPARβ/δ, and hPPARγ systems. NA: not active. | | | | | | |

### Example 4. Cytotoxicity of compounds of Formula I and II

The potential cytotoxic effects of the compounds of the invention **5a**, **5b**, **8a**, and **8b** on the human macrophage cell line THP-1 were evaluated by flow cytometry to assess cell survival using an Annexin V-FITC/PI dual staining assay. The THP-1 cells were treated with concentrations of 30 and 100 µM for 24 h. No cytotoxic effects were observed at any of the tested concentrations (**Figure 1**).

### Example 5. Compounds of Formula I and II regulate lipid metabolism by increasing the expression of Pdk4 in L929sA WT cells

Pyruvate Dehydrogenase Kinase 4 gene (*Pdk4*), a well-known target gene of PPARs, encodes a protein that regulates the phosphorylation and inactivation of the pyruvate dehydrogenase complex in response to physiological conditions. This response switches the energy source from glucose to fatty acids to maintain blood glucose levels to, thereby, increase lipid metabolism. Because it is a well-known target gene of PPARs, the effect of the 2- and 3-prenylated esters of tetrahydroquinolines and quinolines on a *Pdk4* region reported to bind PPARα, was evaluated. The L929sA cells that were stably transfected with a PDK4-(PPRE)₃-Luc+ reporter gene construct were used. Cells were treated with tetrahydroquinolines **5a** and **5b** or quinolines **8a** and **8b** at 10 µM for a total induction time of 6 h, and compared to GW7647 at 1 µM as the positive control (EC₅₀ = 1 nM for PPARa; 2.9 µM for PPARβ/δ; 1.3 µM for PPARγ). Efficacy was determined by calculating their maximal transactivation response, which was expressed as a percentage of maximum GW7647 activity. Results revealed that tetrahydroquinolines **5a** and **5b** and quinoline **8a** promote lipid metabolism as they increased the transactivation of the PDK4-Luc+ indicator and the expression of the *Pdk4* gene in L929sA WT cells. In particular, tetrahydroquinoline **5a** outperformed the maximal activity of the prior art compound GW7647. These findings were subsequently validated by RT-qPCR to assess the expression of *Pdk4* mRNA in the L929sA WT cells treated with tetrahydroquinolines **5a** and **5b** as representative leads of pan-PPAR and selective PPARα agonism, respectively. As shown in **Figure 2B**, the treatment with both **5a** and **5b** at 10 µM led to increased expression levels of *Pdk4.* Collectively, these results show that tetrahydroquinolines **5a** and **5b** promote lipid metabolism, which make them excellent candidates for prevention and/or treatment of lipid-related diseases.

### Example 6. Compounds of Formula I and II minimize inflammation by decreasing TNFα-induced NF-κB transactivation and inhibiting TNFα-induced pro-inflammatory gene expression in L929sA cells

PPARs play crucial roles in controlling inflammatory responses. NF-κB is a transcription factor that plays a pivotal role in the expression of multiple genes involved in the immune and inflammatory responses. To study the potential anti-inflammatory effects of the tetrahydroquinolines and quinolines of the invention, the *NF*-*κB*-dependent physiological human IL-8 promoter construct, p1481.IL8P-Luc+, which is stably integrated into L929sA cells, was tested. After transfection, cells were treated at 10 µM with **5a**, **5b**, **8a**, or **8b** prior to TNFa stimulation. **Figure 3A** shows that tetrahydroquinolines **5a** and **5b** and quinoline **8b** can repress the transactivation of the TNFα-dependent *NF*-*κB*-driven reporter, compared to the prior art compound GW7647. Given the significant decrease in NF-κB-IL8P-Luc+ reporter gene activity observed after treatment with the 2- or 3-prenylated tetrahydroquinolines **5a** and **5b** and the 3-prenylated quinoline **8b**, their potential anti-inflammatory effects at the transcriptional level for other inflammatory mediators was evaluated. To achieve this, the L929sA WT cells were treated with **5a**, **5b** and **8b** at 10 µM for 1 h, followed by TNFa stimulation for a total induction time of 6 h. The expression of inflammatory genes was evaluated by RT-qPCR. Interestingly, tetrahydroquinolines **5a** and **5b** and quinoline **8b** repressed the transactivation of *NF*-*κB*-TNFα-dependent Luc+ and downregulated the transcription of TNFα-induced proinflammatory genes such as Cc/5 and *II-6* in L929sA WT cells (**Figure 3B**).

### Example 7. Tetrahydroquinoline compound 5a improved lipid and carbohydrate metabolism in oblob mice

Tetrahydroquinoline **5a** administered for 15 days (orally, 10 mg/kg/d) to an obese and diabetic mouse model (ob/ob), improved lipid and carbohydrate parameters. Indeed, **5a** decreased total cholesterol and non-HDL cholesterol levels, as well as insulin levels and the HOMA-IR index in the blood of *oblob* mice, compared to vehicle-treated mice (**Figures 4A-4D**). Furthermore, as hepatotoxicity has been identified as an adverse effect of some thiazolidinediones, it is noteworthy that the treatment with tetrahydroquinoline **5a** did not increase levels of hepatic transaminases such as ALT and AST (**Figures 4E-4F**).

### Example 8. Tetrahydroquinoline compound 5a increased the expression of genes of fatty acid use and oxidation in liver and fat of ob/ob mice

Tetrahydroquinoline **5a** also increased the expression of genes involved in mitochondrial and peroxisomal β-oxidation of fatty acids, such as *Pdk4* (pyruvate dehydrogenase kinase *4), Cpt1a* (carnitine palmitoyltransferase 1A) and *Acox1* (acyl-CoA oxidase 1) in epididymal white adipose tissue and/ or liver of *oblob* mice (**Figure 5**). These results indicate that tetrahydroquinoline **5a** exerts a strong activation of the PPARα which might promote lipid metabolism and energy expenditure by increasing fatty acid transport to the mitochondria and oxidation.

### Example 9. Molecular modelling results with tetrahydroquinoline compound 5a

A molecular modelling study of 2-prenylated tetrahydroquinoline **5a** was carried out. For PPARα, the combined analysis, performed using docking calculations and molecular dynamics simulations, predicted that tetrahydroquinoline **5a** bind to the same region of the active site as previously reported for WY-14,643 (Bernardes et al. (2013)). The molecular dynamics simulations indicated that **5a** was spatially arranged in a slightly different manner from that of WY-14,643. The interactions previously reported as the most important for stabilising ligand-receptor complexes are present (Bernardes et al. (2013)). Such interactions occur with the Ser280, Ile317, His440, and Tyr464 residues. However, it is interesting to note that, in general, the interactions observed for 2-prenylated tetrahydroquinoline **5a** were weaker than those noted for WY-14,643. This result is consistent with the experimental results of the previous examples, in which the agonist activity of **5a** was weaker than that of the prior art compound WY-14643.

With the PPARβ/δ receptor, although **5a** was spatially arranged in a similar way to GW501516 (Wu et al. (2017)), the interactions of **5a** were clearly significantly weaker than those observed for the reference compound GW501516. The main interactions were for Cys249, Arg248, Thr253, Ile320, His413 and Lys331.

With the PPARγ receptor, the molecular behaviour of **5a** was compared to that previously reported for rosiglitazone (Kouskoumvekaki et al. (2013)). The molecular dynamics simulations predicted that tetrahydroquinoline **5a** binds to the active site in a similar way to that reported for rosiglitazone. The main interactions were for Cys285, Arg288, Leu330, Ile342, His449 and Tyr473, and the interactions obtained for **5a** were weaker than those observed for rosiglitazone.

### CITATION LIST

Bernardes et al. (2013). Molecular mechanism of peroxisome proliferator-activated receptor α activation by WY14643: a new mode of ligand recognition and receptor stabilization. J Mol Biol.;425(16):2878-93. doi: 10.1016/j.jmb.2013.05.010.
Nolte et al. (1998). Ligand binding and co-activator assembly of the peroxisome proliferator-activated receptor-gamma. Nature.;395(6698):137-43. doi: 10.1038/25931.
Kouskoumvekaki et al. (2013). Discovery of a novel selective PPARγ ligand with partial agonist binding properties by integrated in silico/in vitro work flow. J Chem Inf Model.;53(4):923-37. doi: 10.1021/ci3006148.
Wu et al. (2017). Structural basis for specific ligation of the peroxisome proliferator-activated receptor δ. Proc Natl Acad Sci U S A.;114(13):E2563-E2570. doi: 10.1073/pnas.1621513114.

## Claims

1. A prenylated tetrahydroquinoline compound or prenylated quinoline compound, of Formula I or Formula II, respectively, or a salt, or solvate thereof: 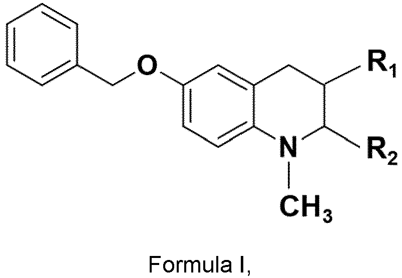 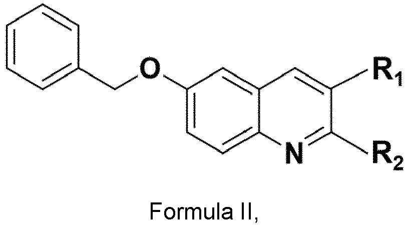 wherein:
- R₁ is independently selected from H or CH₂-CH=C(CH₃)-CH₂-CH₂-COOR₃;
- R₂ is independently selected from CH₃ or CH₂-CH₂-CH=C(CH₃)-CH₂-CH₂-COOR₃;
- R₃ is independently selected from the group consisting of H, alkyl, alkylamide, alkylamine, alkylaryl, alkyl-ether, and acyl; and
wherein:
- when R₁ is H, then R₂ is CH₂-CH₂-CH=C(CH₃)-CH₂-CH₂-COOR₃; and
- when R₂ is CH₃, then R₁ is CH₂-CH=C(CH₃)-CH₂-CH₂-COOR₃.

2. The compound, salt, or solvate according to claim 1, wherein R₃ is independently selected from H or CH₂-CH₃.

3. The compound, salt, or solvate according to claim 1 or 2, wherein said compound is selected from the group consisting of 5a, 5b, 6a, 6b, 8a, 8b, 9a and 9b: 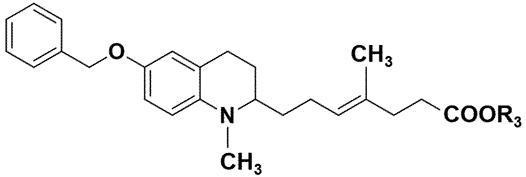
5a: R₃ = CH₂CH₃,
6a: R₃ = H, 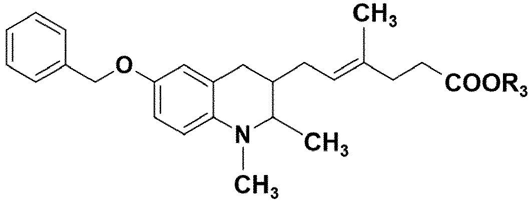
5b: R₃ = CH₂CH₃,
6b: R₃ = H, 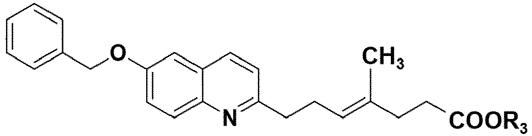
8a: R₃ = CH₂CH₃,
9a: R₃ = H, 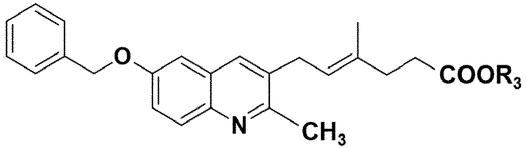
8b: R₃ = CH₂CH₃,
9b: R₃ = H.

4. A pharmaceutical composition comprising at least a compound, salt, or solvate according to any one of claims 1 to 3 as active ingredient, and at least a pharmaceutically acceptable excipient or carrier.

5. The pharmaceutical composition according to claim 4, further comprising at least one additional active ingredient.

6. The compound, salt, or solvate according to any one of claims 1 to 3, or the pharmaceutical composition according to claim 4 or 5, for use as a medicament.

7. The compound, salt, or solvate according to any one of claims 1 to 3, or the pharmaceutical composition according to claim 4 or 5, for use in the prevention and/or treatment of a peroxisome proliferator-activated receptor (PPAR)-mediated disease.

8. The compound, salt, or solvate or pharmaceutical composition for use according to claim 6 or 7, wherein said compound is an agonist of at least one PPAR protein, wherein said PPAR protein is selected from the group consisting of PPARα, PPARβ/δ and PPARγ.

9. The compound, salt, or solvate, or pharmaceutical composition for use according to any one of claims 6 to 8, wherein said compound is a dual PPARα/γ agonist.

10. The compound, salt, or solvate, or pharmaceutical composition for use according to any one of claims 6 to 8, wherein said compound is an agonist of PPARα, PPARβ/δ and PPARγ.

11. The compound, salt, or solvate, or pharmaceutical composition for use according to any one of claims 7 to 10, wherein said disease is selected from the group consisting of PPARα-mediated disease, PPARβ/δ-mediated disease, and PPARγ-mediated disease.

12. The compound, salt, or solvate, or pharmaceutical composition for use according to any one of claims 7 to 11, wherein said disease is associated to the expression of a gene selected from the group consisting of *Pdk4*, *NF-κB, CcI2, CcI5*, *Il6*, *Tnf, Cpt1a,* and *Acox1.*

13. The compound, salt, or solvate, or pharmaceutical composition for use according to any one of claims 7 to 12, wherein said disease is associated with inflammation.

14. The compound, salt, or solvate, or pharmaceutical composition for use according to claim 13, wherein said inflammation is selected from autoimmune inflammation or neuroinflammation.

15. The compound, salt, or solvate, or pharmaceutical composition for use according to any one of claims 7 to 14, wherein said disease is selected from the group consisting of metabolic syndrome, type 2 diabetes mellitus, dyslipidemia, hyperlipidemia, hypertriglyceridemia, hypercholesterolemia, obesity, dyslipidemic atherosclerosis, metabolic dysfunction-associated fatty liver disease (MAFLD), cardiovascular disease, cardiometabolic disease, neurodegenerative disease, Friedreich's ataxia, Parkinson's disease, multiple sclerosis, Alzheimer's disease, autoimmune disease, rheumatoid arthritis, autoimmune thyroid disease, dermatological disease, and cancer.
